# EUROPEAN PATENT SPECIFICATION

(11) **EP 3 768 079 B1**
(45) Date of publication and mention of the grant of the patent: **29.04.2026**
(21) Application number: 19770329.1
(22) Date of filing: 05.03.2019
(51) Int. Cl.: A01K 67/0278, C07K 16/00, C07K 16/46

(54) **TRANSGENIC CHICKEN THAT PRODUCES HUMAN ANTIBODIES**
TRANSGENES HUHN, DAS MENSCHLICHE ANTIKÖRPER PRODUZIERT
POULET TRANSGÉNIQUE QUI PRODUIT DES ANTICORPS HUMAINS

(30) Priority: 21.03.2018 US 201862646319 P
(43) Date of publication of application: 27.01.2021
(73) Proprietor: Crystal Bioscience Inc., San Diego, CA 92121 (US)
(72) Inventor: LEIGHTON, Philip A., San Diego, California 92121 (US); HARRIMAN, William Don, San Diego, California 92121 (US)
(74) Representative: AWA Denmark A/S
(86) International application number: PCT/US2019/020799
(87) International publication number: WO 2019/182751

(56) References cited:
- WO-A1-2011/019844
- US-A1- 2004 250 307
- US-A1- 2013 261 012
- US-A1- 2014 155 580
- US-A1- 2015 307 595
- US-A1- 2017 156 298
- US-A1- 2017 181 414
- BENJAMIN SCHUSSER ET AL: "Harnessing Gene Conversion in Chicken B Cells to Create a Human Antibody Sequence Repertoire", PLOS ONE, vol. 8, no. 11, 21 November 2013 (2013-11-21), pages 1 - 15, XP055096053, DOI: 10.1371/journal.pone.0080108
- KATHRYN H. CHING ET AL: "Chickens with humanized immunoglobulin genes generate antibodies with high affinity and broad epitope coverage to conserved targets", MABS, vol. 10, no. 1, 2 November 2017 (2017-11-02), US, pages 71 - 80, XP055525565, ISSN: 1942-0862, DOI: 10.1080/19420862.2017.1386825
- KARL ROSNER ET AL: "Third complementarity-determining region of mutated VH immunoglobulin genes contains shorter V, D, J, P, and N components than non-mutated genes", IMMUNOLOGY, vol. 103, 1 January 2001 (2001-01-01), pages 179 - 187, XP055516887
- WU LEEYING ET AL: "Fundamental Characteristics of the Immunoglobulin V H Repertoire of Chickens in Comparison with Those of Humans, Mice, and Camelids", vol. 188, no. 1, 1 January 2012 (2012-01-01), US, pages 322 - 333, XP055854741, ISSN: 0022-1767, Retrieved from the Internet <URL:https://www.jimmunol.org/content/jimmunol/188/1/322.full.pdf> DOI: 10.4049/jimmunol.1102466
- PHILIP A. LEIGHTON ET AL: "V(D)J Rearrangement Is Dispensable for Producing CDR-H3 Sequence Diversity in a Gene Converting Species", FRONTIERS IN IMMUNOLOGY, vol. 9, no. 1317, 11 June 2018 (2018-06-11), pages 1 - 14, XP055669112, DOI: 10.3389/fimmu.2018.01317
- BERENS ET AL.: "Use of a single VH family and long CDR3s in the variable region of cattle Ig heavy chains", INTERNATIONAL IMMUNITY, vol. 9, no. 1, 31 January 1997 (1997-01-31), pages 189 - 199, XP055637599

## Description

### CROSS-REFERENCING

This application claims the benefit of U.S. provisional application serial no. 62/646,319, filed on March 21, 2018.

### BACKGROUND

As in all higher vertebrates, a critical checkpoint in B cell development in chickens is in-frame V(D)J rearrangement, leading to expression of a functional B cell receptor complex at the cell surface (1-3). The process of rearrangement in chickens utilizes the same recombination signal sequences and enzymes as in mammals, recombining V, D and J genes into functional V regions (4-8). The main difference in chickens is that in both the light and heavy chain loci, there is only a single germline V gene and a single germline J gene, and in the heavy chain, a cluster of highly similar D segments (5,8), rather than the large number of diverse V, D and J genes in humans. The rearrangement process thus produces very little sequence diversity in the initial B cell repertoire. Imperfect joins and exonucleolytic chewing back in the V-J and V-D-J junctions can generate some diversity, but chicken B cells do not express TdT (9), so there are no N-additions in CDR-H3 and in general, the immunoglobulin diversity produced by the gene rearrangement process is minimal (5,10,11).

To produce a diverse repertoire, chickens employ a process of gene conversion in which upstream pseudogenes in the light and heavy chain loci serve as sequence donors to mutate the expressed, functional V (8,11-13). These pseudogenes do not contain promoters or recombination signal sequences, so they cannot be expressed themselves, but their sequences are incorporated in segments of varying length into the single functional V. Multiple rounds of overlapping gene conversion from different pseudogenes in the pool lead to a highly diverse naive repertoire. In addition to gene conversion, non-templated somatic hypermutation also contributes to repertoire diversity (14-16). Despite the limitations of V(D)J rearrangement in chickens, CDR-H3s exhibit length and sequence diversity comparable to that in mammals (1,17). The function of the chicken Ds in V(D)J rearrangement may be more related to providing intra-CDR-H3 disulfide bridges for stabilization of antigen-binding loops (17), since most Ds encode a single cysteine residue and D-D joins would thus encode paired cysteines. Diversity in chicken CDR-H3s comes from gene conversion/somatic hypermutation, rather than the rearrangement process itself.

This disclosure provides a transgenic chicken that produces human antibodies.

### SUMMARY

The invention is set out as in the appended set of claims and provides, among other things, a transgenic chicken that has a modified immunoglobulin heavy chain immunoglobulin locus. The modified locus has no endogenous V-D-J region and, instead, has a human VH segment, a human D cluster, a human J segment and a plurality of upstream pseudogenes based on human V_{H} sequences. The modified IgH locus undergoes V(D)J recombination in the chicken, gene conversion between the upstream pseudogenes and the human VH segment occurs, and the chicken produces antibodies that have a diversified immunoglobulin heavy chain, include CDR3s of differing lengths.

The present transgenic chicken may have certain advantages over other transgenic chickens that are designed to produce recombinant antibodies. For example, the IgH locus of the present transgenic chicken lacks the endogenous V-D-J region, thereby preventing the human sequences from being removed during V(D)J recombination (by, e.g., recombination between a chicken D and a human J). Moreover, in mouse, the V-D-J region contains genes that are essential for fertility (i.e., the adam6a and adam6b genes, see, e.g., Marcello et al J. Biol. Chem. 2011 286: 13060-70 as well as US8,697,940). The complete sequence of the endogenous V-D-J region in the chicken genome is currently unknown. As such, it was not clear whether the endogenous V-D-J region in the chicken genome contains essential genes, or a chicken that contains an IgH locus that lacks the entire contiguous endogenous chicken V-D-J region could be made. Further, the transgenic chicken produces antibodies that have a diversified immunoglobulin heavy chain, where the combination of V(D)J recombination, gene conversion, and somatic hypermutation contribute to the diversification. Finally, because (a) a significant amount of diversity in the heavy chain CDR3 is accomplished via V(D)J recombination (as opposed to solely gene conversion) and (b) the lack of terminal transferase in chicken limits the lengths of the CDRs to what is encoded by the D genes, the antibodies produced by the chicken are shorter and can be thought of as "more human" than chicken antibodies (which typically have a longer heavy chain CDR3 because of D-D joining, which is selected for in chickens).

In wild type chickens, longer CDR-H3s are produced by tandem D-D joining, and also probably by gene conversion, which can be used to insert sequences, delete sequences as well as just change sequences. Thus, even without terminal transferase, the CDR-H3s can still be long. In the present chicken, the CDR-H3s are shorter than the CDR-H3s of wild type chickens and humans, and shorter than the theoretical range of 16-23 amino acids that would occur by simple joining of V, D and J with no chewing back, suggesting that there is no D-D joining or other mechanism to create long CDRs in the present chicken. Comparing antibodies made from the present chicken to antibodies from humans, the CDR-H3s are shorter in the present chickens (average length = 12, versus 15-16 in humans).

Also provided are methods of producing and method of using the transgenic chicken.

### BRIEF DESCRIPTION OF THE DRAWINGS

Some aspects of the present invention may be best understood from the following detailed description when read in conjunction with the accompanying drawings. It is emphasized that, according to common practice, the various features of the drawings are not to scale. Indeed, the dimensions of the various features are arbitrarily expanded or reduced for clarity. Included in the drawings are the following figures.

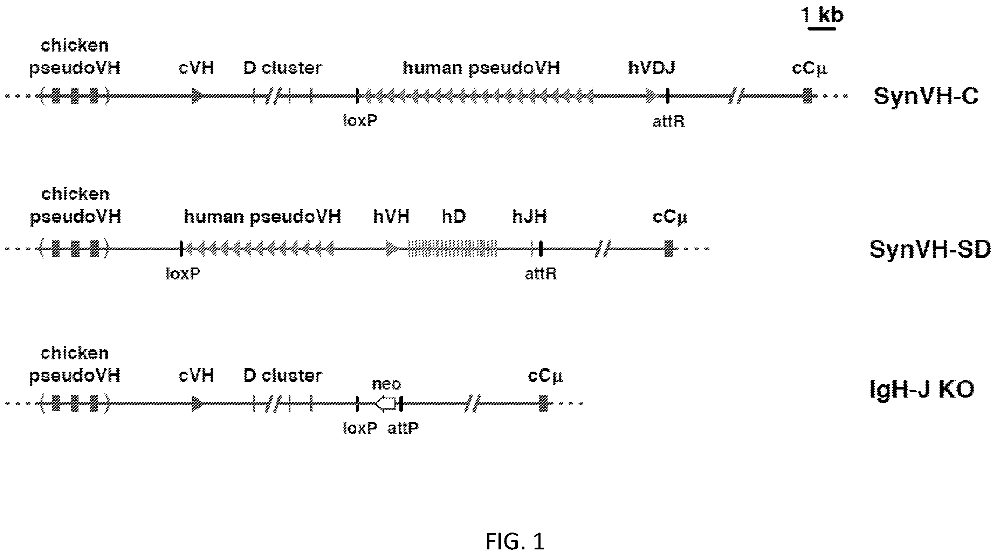

**FIG. 1** provides diagrams of pre-rearranged and rearranging human VH segments in the chicken. Scale diagrams of SynVH-C (pre-rearranged) and SynVH-SD (rearranging) transgenes and the heavy chain knockout (IgH-J KO). Human sequences are shown in red and chicken in blue. Top line, the SynVH-C transgene consists of a pre-rearranged human V region (hVDJ) with an upstream array of human pseudogenes. The human V region splices to the downstream chicken constant regions (only Cmu is shown). The chicken germline V and D genes, and pseudogene array, are upstream of the human V genes. Precise mapping of chicken pseudogenes is not shown (as indicated by parentheses), but the distance to the functional chicken V is accurate. Remnant loxP and attR sites from the insertion event are shown. Middle line, the SynVH-SD transgene contains a single human germline VH3-23 gene, single JH6 gene, and 24 unique human Ds. All intervening sequences and recombination sites are from the chicken heavy chain locus (shown in blue). Upstream of the human germline V gene is an array of human-based pseudogenes. The upstream chicken germline V and D genes are deleted, but the chicken pseudogenes are still present.
   Bottom line, structure of the chicken heavy chain knockout (21). The genotype of the transgenic chickens in this study was SynVH-C or SD "knock-in"/IgH knockout. The single chicken JH gene was replaced with a promoterless neo gene. The attP site adjacent to the neo gene is where the SynVH-C and SynVH-SD constructs were inserted, followed by Crelox recombination to remove selectable markers and plasmid backbone sequences.

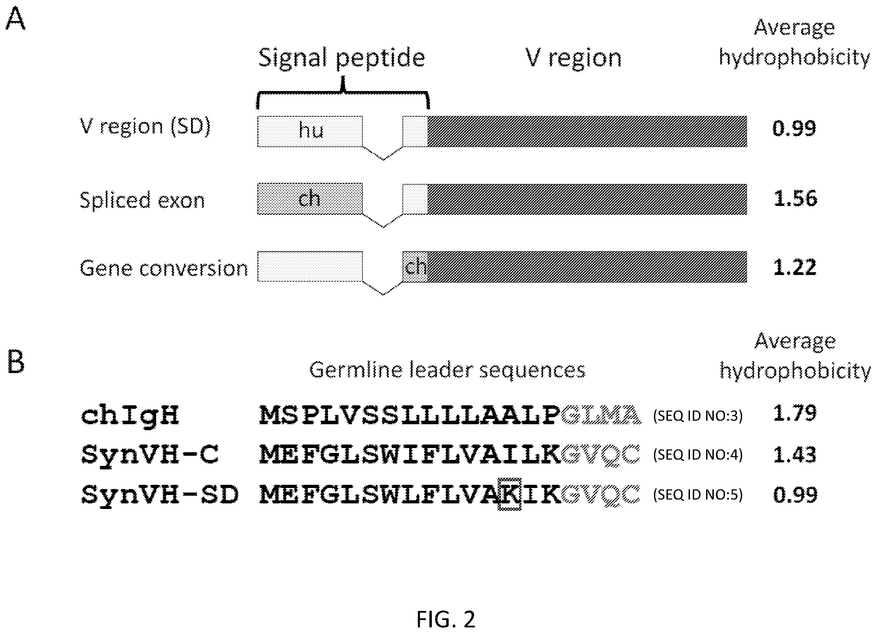

**FIG. 2****,** A-B provides a diagram of signal peptide changes observed in transgenic chickens and an alignment of germline signal peptide sequences. The SynVH-SD signal peptide is less hydrophobic than that of SynVH-C and undergoes changes to become more hydrophobic. A. Diagram of the signal peptide changes observed in transgenic chickens. The signal peptide can be changed to chicken sequences either by splicing of the chicken signal peptide exon to the human V region exon, or from gene conversion of the portion in the V region exon. Human signal peptide is shown in yellow, chicken in orange. The average hydrophobicity (Kyte-Doolittle, without normalization) is shown for each type of signal peptide. B. Alignment of the germline signal peptide sequences from the WT chicken VH segment, SynVH-C and SynVH-SD. The four amino acids encoded in the V region exon are in red. The lysine residue that is often mutated to a hydrophobic residue in SynVH-SD is boxed. The average hydrophobicity is indicated at right.

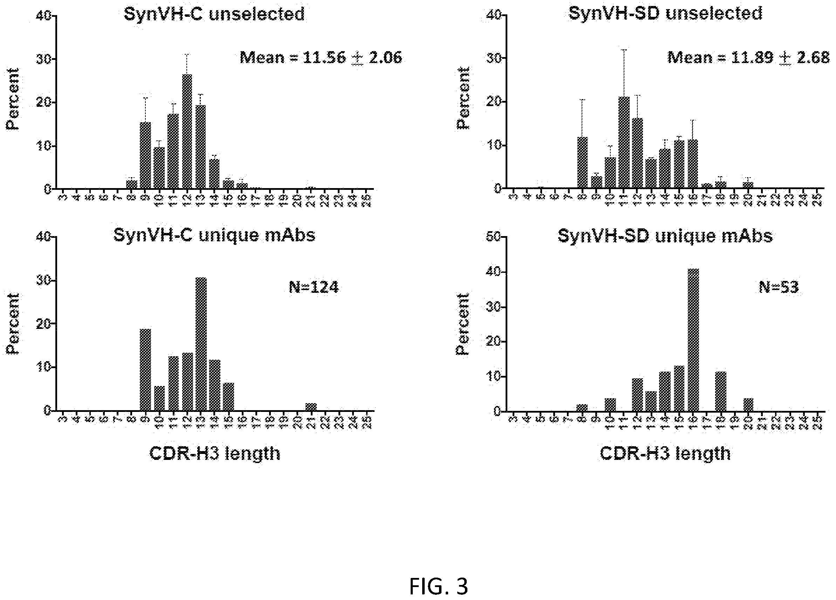

**FIG. 3** provides CDR-H3 lengths. CDR-H3 lengths show a broader distribution in SynVH-SD. CDR-H3 lengths from all of the sequences ("unselected", top two panels) were calculated based on IMGT positions 105-117 and plotted based on the frequency of each length out of all sequences with a clear CDR-H3 (N=3,099,355 for SynVH-C, N=1,050,398 for SynVH-SD). Error bars indicate bird-to-bird variation; SynVH-C, N=6 birds, SynVH-SD, N=3 birds. Mean and SD are given. Lower two panels show CDR-H3 lengths of the PGRN-specific mAbs.

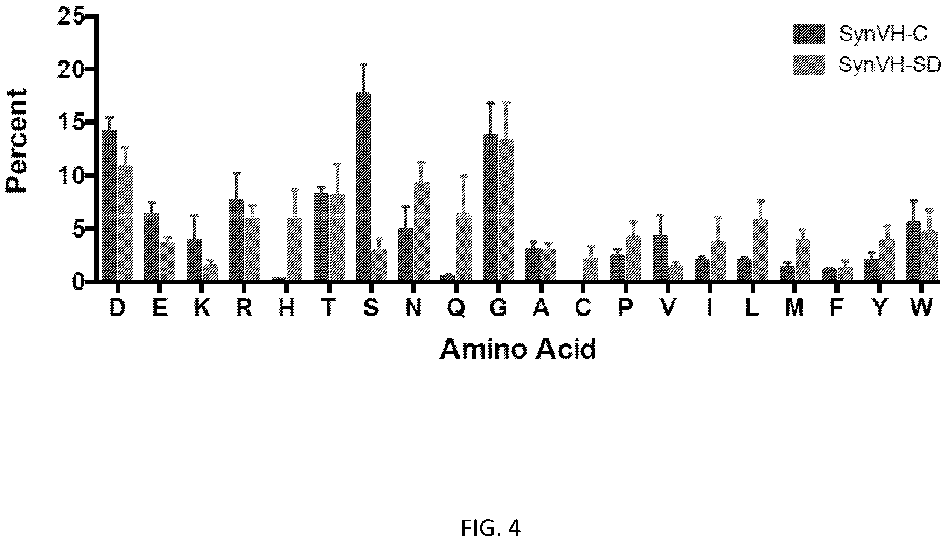

**FIG. 4** provides SynVH-C and SynVH-SD amino acid frequencies. SynVH-C and SynVH-SD amino acid frequencies in CDR-H3 are similar for most amino acids. The frequency of each amino acid out of the total amino acid content in CDR-H3, in the positions not contributed by the V or J genes (107-109 or 111). Specific CDR-H3 lengths of 12, 13, 14 and 15 residues from the top 1000 unique sequences from each bird were used to calculate the frequencies, which were then averaged. Error bars indicate bird-to-bird variation; SynVH-C, N=6 birds, SynVH-SD, N=3 birds.

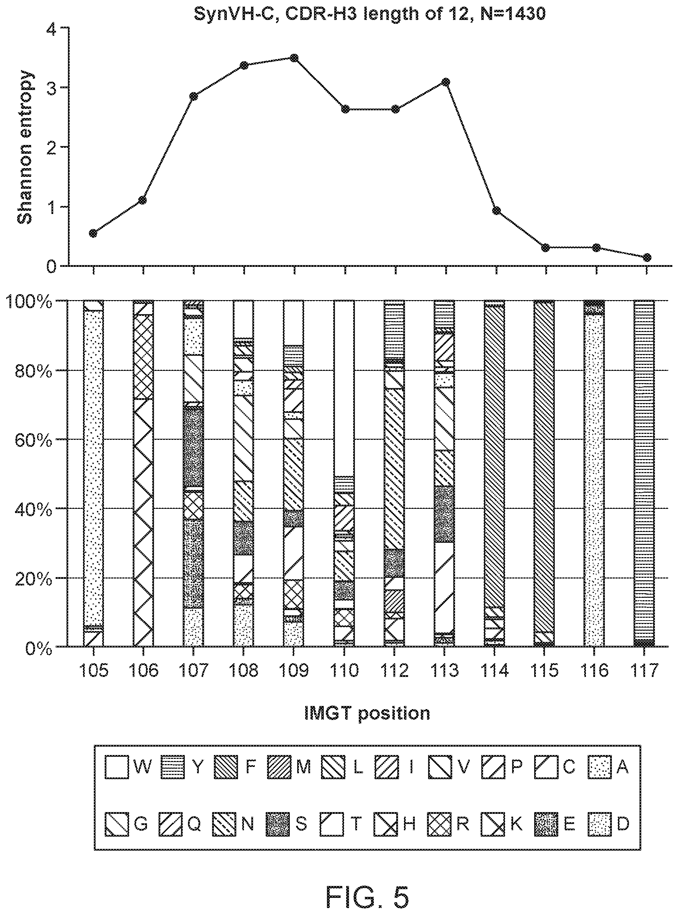

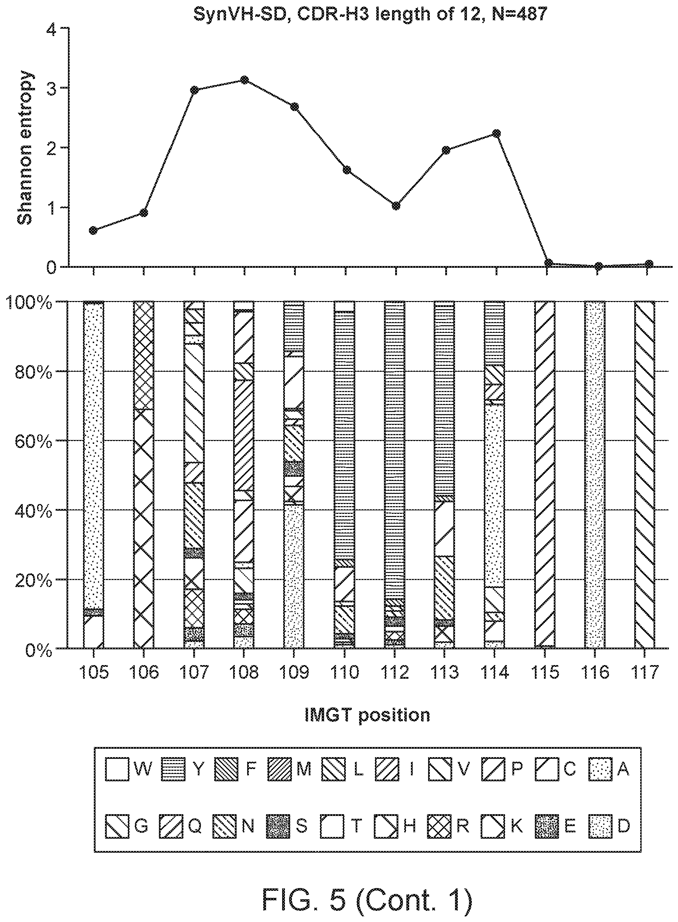

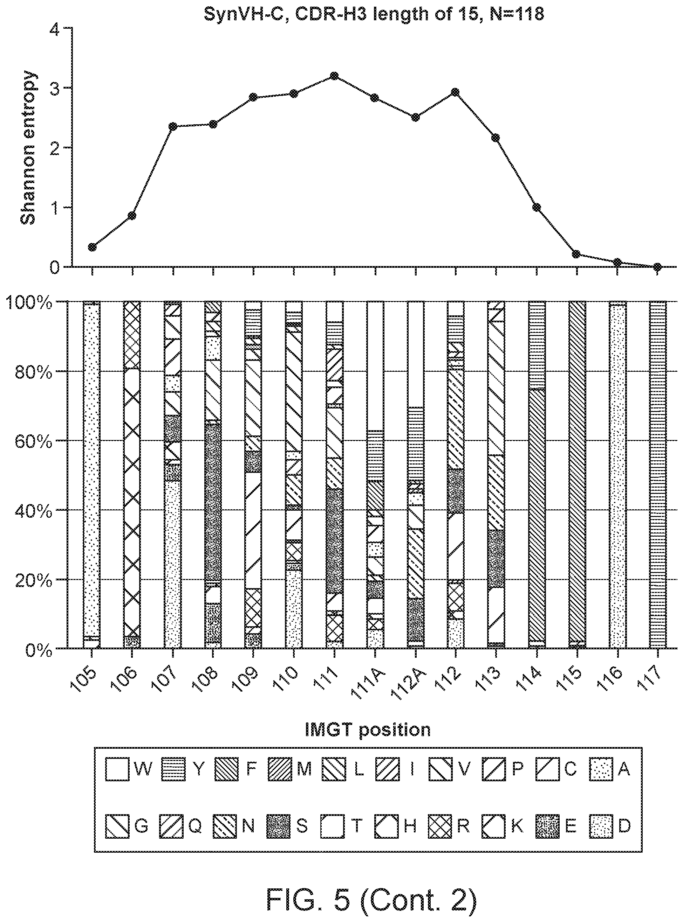

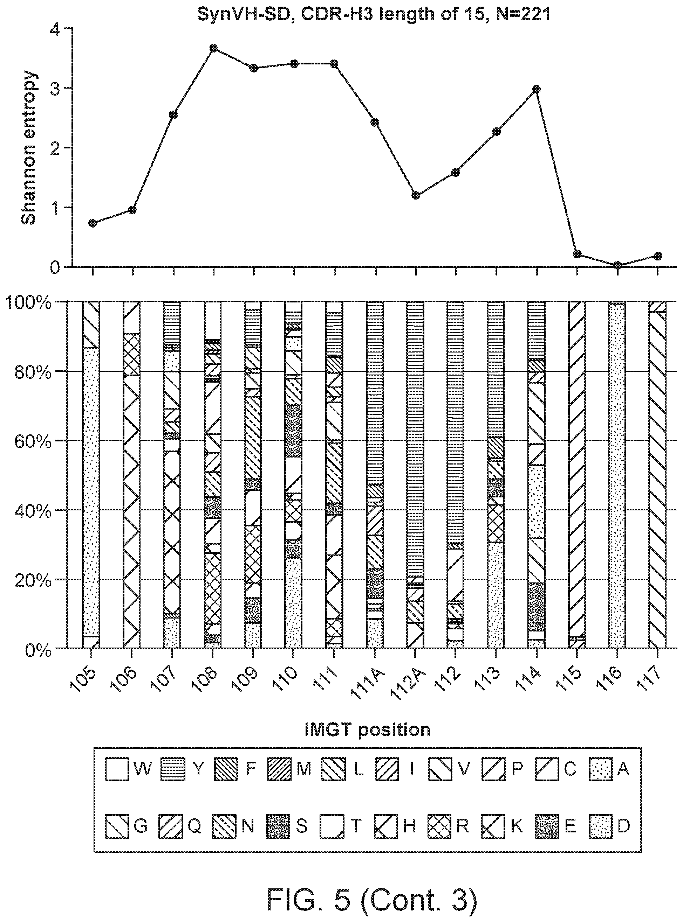

FIG. 5 depicts sequence diversity in CDR-H3 in SynVH-C and SynVH-SD.
   Sequence diversity in CDR-H3 is similar in SynVH-C and SynVH-SD. Shannon entropy and amino acid distribution for each position of CDR-H3 IMGT positions 105-117 are shown for the specific lengths of 12 residues (top two panels) and 15 residues (bottom two panels) for SynVH-C and SynVH-SD. The sequences were drawn from the top 1000 sequences from each bird; SynVH-C, 6 birds, SynVH-SD, 3 birds.

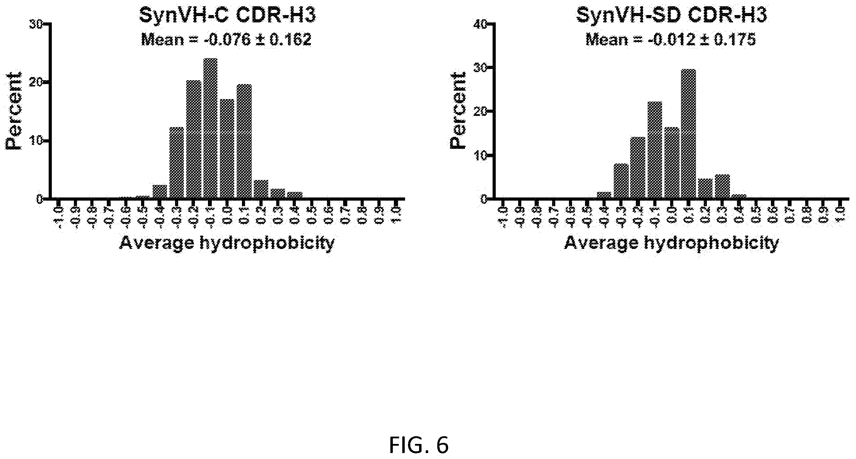

**FIG. 6** depicts hydrophobicity of CDR-H3 in synVH-C and SynVH-SD.
   Hydrophobicity of CDR-H3 is similar in SynVH-C and SynVH-SD. Average hydrophobicity based on the normalized Kyte-Doolittle scale (34,35) of CDR-H3s of lengths 12-15 residues (IMGT positions 105-117) were calculated for the top 1000 sequences from each bird. The frequencies of each hydrophobicity value (grouped into increments of 0.1) are shown in the graphs.

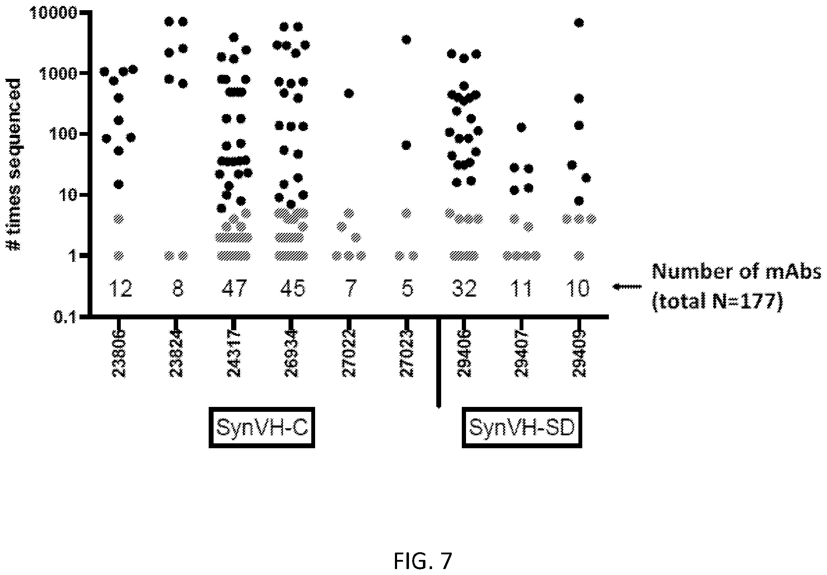

**FIG. 7** depicts the frequency of mAb sequences in NGS data. The frequency of mAb sequences in NGS data show that many mAbs identified by GEM screening are rare in the spleen population. Number of times each antigen-specific mAb sequence identified in GEM screens was found in all of the sequencing data from each bird. The mAbs are grouped by bird and transgene, either SynVH-C or SynVH-SD, as indicated. The number of mAbs from each bird is indicated below the plots. mAbs that were sequenced 5 or fewer times in the whole data set are colored red, showing that rare mAbs are found in the GEM screens.

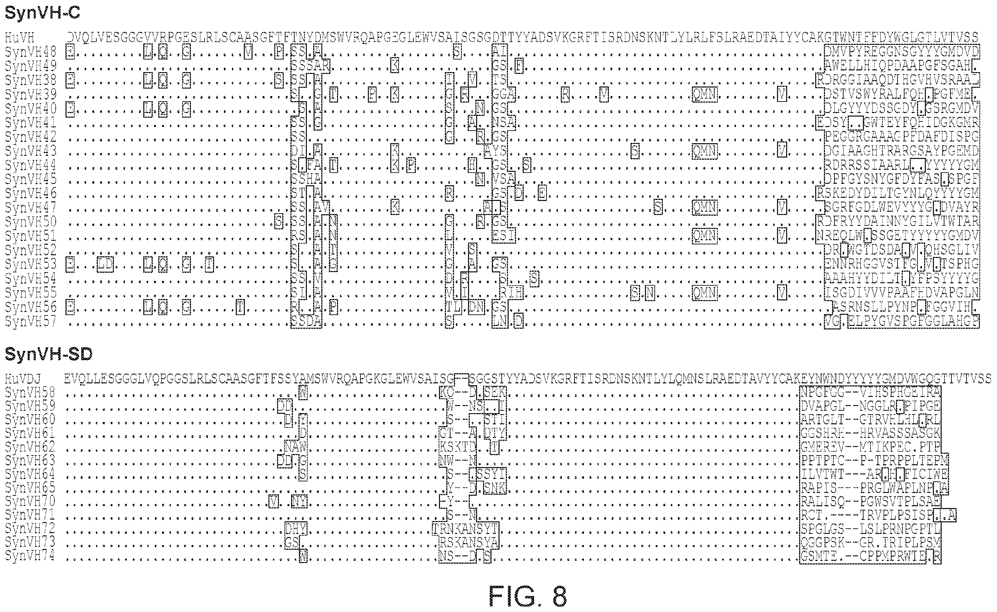

**FIG. 8** depicts alignments of human designed pseudogenes with the functional V sequences in each construct. For SynVH-C, diverse CDR-H1, 2 and 3 sequences were obtained from the NIH EST database queried with the human VH3-23 gene. The germline VH3-23 framework sequence was included in some of the pseudogenes, whereas others match the functional VH sequence (HuVH, top line) which is a somatically-derived sequence with 9 changes relative to the germline. For SynVH-SD, CDR-H1 and 2 were derived from the human VH3 family members, and place on the VH3-23 framework scaffold. No specific CDR-H3 sequences were included; the sequences shown are the spacers placed between pseudogenes. (HuVH is set forth in SEQ ID NO: 6; SynVH48 is set forth in SEQ ID NO: 7; SynVH49 is set forth in SEQ ID NO: 8; SynVH38 is set forth in SEQ ID NO: 9; SynVH39 is set forth in SEQ ID NO: 10; SynVH40 is set forth in SEQ ID NO: 11; SynVH41 is set forth in SEQ ID NO: 12; SynVH42 is set forth in SEQ ID NO: 13; SynVH43 is set forth in SEQ ID NO: 14; SynVH44 is set forth in SEQ ID NO: 15; SynVH45 is set forth in SEQ ID NO: 16; SynVH46 is set forth in SEQ ID NO: 17; SynVH47 is set forth in SEQ ID NO: 18; SynVH50 is set forth in SEQ ID NO: 19; SynVH51 is set forth in SEQ ID NO: 20; SynVH52 is set forth in SEQ ID NO: 21;
   SynVH53 is set forth in SEQ ID NO: 22; SynVH54 is set forth in SEQ ID NO: 23; SynVH55 is set forth in SEQ ID NO: 24; SynVH56 is set forth in SEQ ID NO: 25; SynVH57 is set forth in SEQ ID NO: 26; HuVDJ is set forth in SEQ ID NO: 27; SynVH58 is set forth in SEQ ID NO: 28; SynVH59 is set forth in SEQ ID NO: 29; SynVH60 is set forth in SEQ ID NO: 30; SynVH61 is set forth in SEQ ID NO: 31; SynVH62 is set forth in SEQ ID NO: 32; SynVH63 is set forth in SEQ ID NO: 33; SynVH64 is set forth in SEQ ID NO: 34; SynVH65 is set forth in SEQ ID NO: 35; SynVH70 is set forth in SEQ ID NO: 36; SynVH71 is set forth in SEQ ID NO: 37; SynVH72 is set forth in SEQ ID NO: 38; SynVH73 is set forth in SEQ ID NO: 39; SynVH74 is set forth in SEQ ID NO: 40)

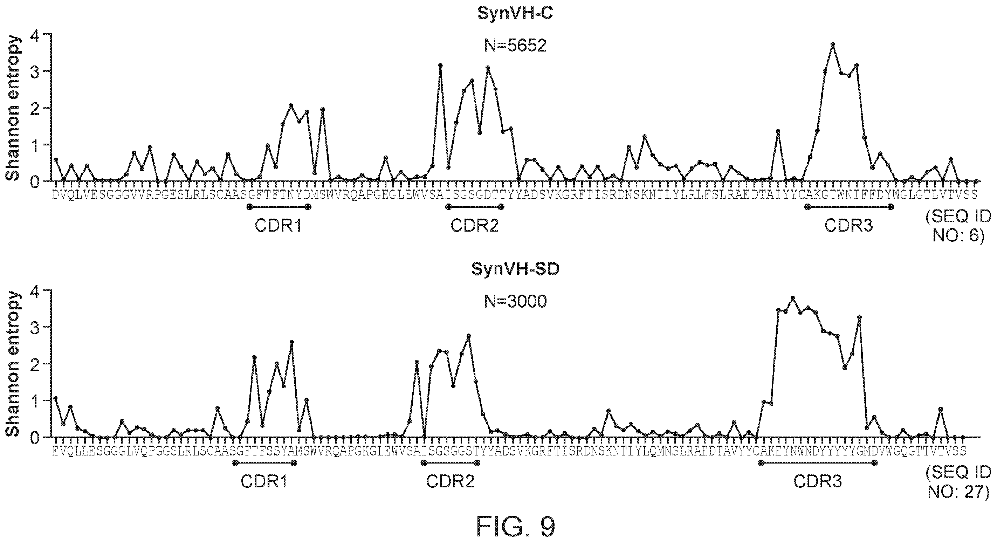

**FIG. 9** depicts Shannon entropy for all of the aligned V region sequences from SynVH-C and SynVH-SD. The top 1000 sequences from each bird were included. CDR designations (IMGT) are indicated.

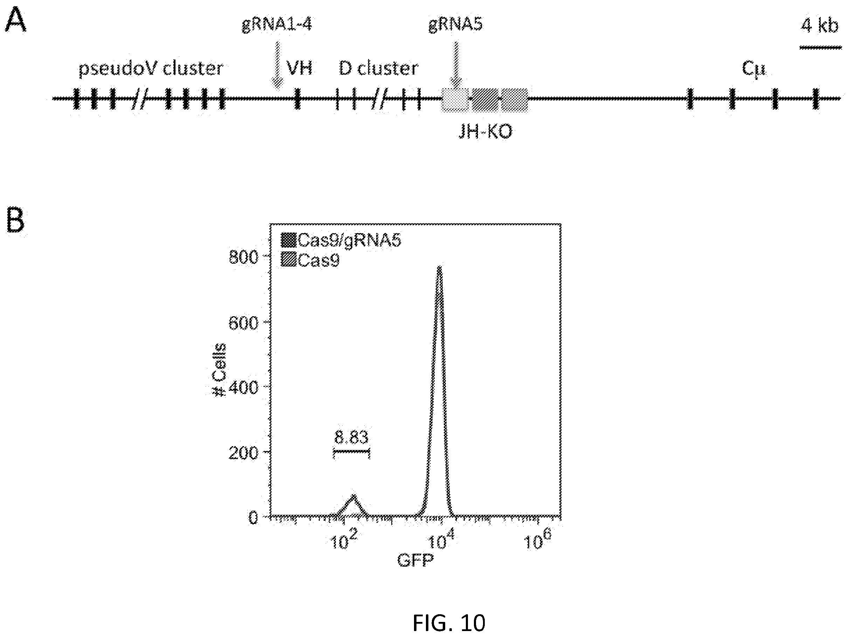

**FIG. 10** Strategy for CRISPR targeting. A. Diagram of the chicken IgH locus present in PGC line 472-138 used for CRISPR targeting. The IgH locus contained a previously obtained knockout of the JH gene segment (JH-KO), between the D cluster and the constant regions (only Cµ is shown), which was replaced with a selectable marker cassette. gRNAs 1 through 4 were designed to target a region upstream of the single functional VH region (indicated with an arrow), and gRNA5 was designed to target the EGFP gene. B. PGCs of line 472-138 were transiently transfected with a construct containing Cas9 or Cas9/gRNA5, specific for EGFP. After 9 days in culture, the cells were analyzed by flow cytometry for loss of green fluorescence.

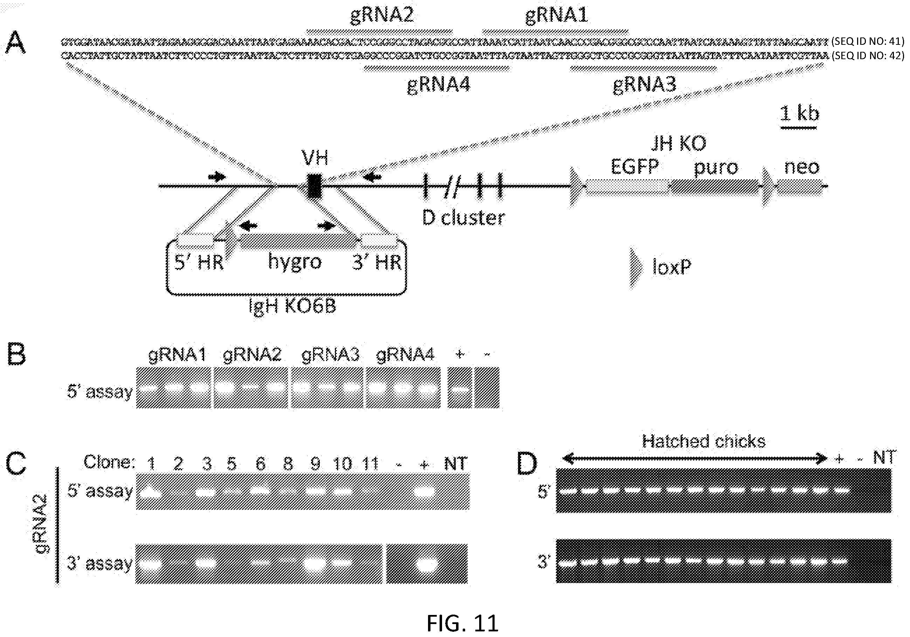

**FIG. 11** CRISPR-mediated targeting of IgH KO6B in PGCs. A. Detailed diagram of the IgH locus. The 122 bp sequence between the 5' and 3' homology regions in IgH KO6B, used to design the gRNAs, is shown at top. The locations of gRNAs 1-4 are indicated with blue lines above the sequence and the protospacer adjacent motifs (PAM) indicated with red lines. The repair vector IgH KO6B (below) contains 5' and 3' homology regions (HR) in yellow, a single loxP site (blue arrowhead), and a hygromycin selection cassette (orange). The locations of the primer binding sites for the 5' and 3' targeting assays are shown as black arrows. The downstream selectable markers in the JH-KO consist of floxed EGFP (green box) and puro gene (blue box), and a promoterless neo gene in opposite orientation (pink box). The loxP sites are blue arrowheads. B. The 5' targeting assay performed on independent, non-clonal cell populations obtained from co-transfection of the four different gRNAs into 472-138 cells along with Cas9 and IgH KO6B. For each gRNA transfection, 3 hygromycin-resistant populations were analyzed. The positive control (+) was a DT40 cell line that contained a knockout of the functional V region [22] and the negative control (-) was the parental IgH KO6B plasmid. C. The 5' and 3' targeting assays performed on 9 independent clones obtained with gRNA2 (there were 12 clones, but clones 4, 7 and 12 grew more slowly and were not tested at this time). Variation in band intensity is likely to be from variation in the template gDNA amount, since the number of cells harvested was not normalized. The negative control (-) was genomic DNA from a JH-KO transgenic bird, and the positive control (+) was a pool of cells (G2) from the gRNA2 experiment in B. NT, no template control. D. The same 5' and 3' targeting assays performed on EGFP+ birds obtained from breeding cell line 1783-10 chimeras to wild type.

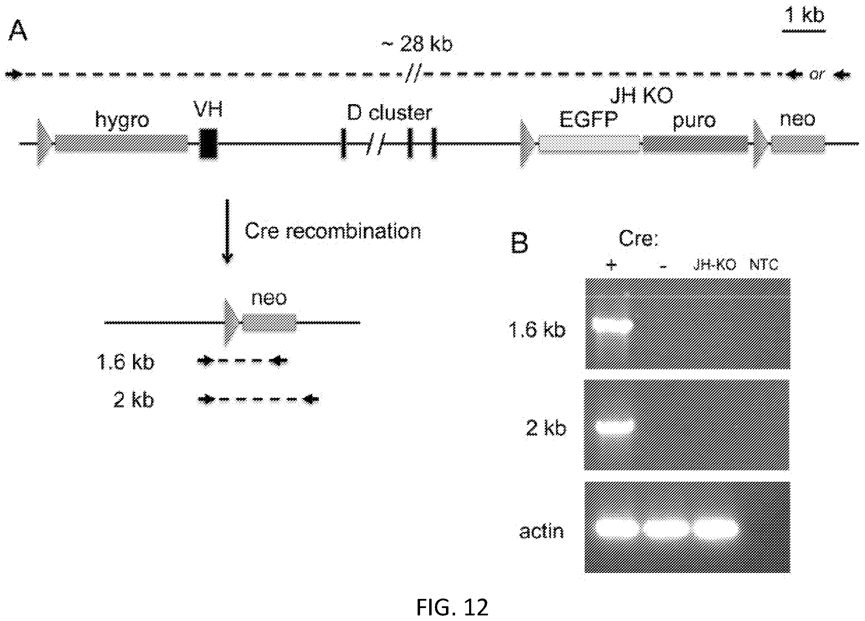

**FIG. 12** Cre recombination of CRISPR-targeted loxP site. A. Diagram of the targeted IgH locus before and after Cre recombination. A forward primer upstream of the CRISPR-targeted loxP site was used with two different reverse primers downstream of the loxP site in the JH-KO cassette. In the nonrecombined allele, the forward and reverse primers are separated by about 28kb on the chromosome. After Cre recombination, a single loxP site and the promoterless neo gene remain, and the primers are either 1.6 or 2kb apart, which amplifies readily. B. PCR of recombined cells. Cre +: gDNA template from 1783-9 cells transfected with Cre; Cre -, parental 1783-9 cells; JH-KO, gDNA from a heterozygous JH-KO bird; NTC, no template control.

### DEFINITIONS

The phrase "transgenic chicken" refers to an animal comprising cells containing foreign nucleic acid (i.e., recombinant nucleic acid that is not native to the chicken). The foreign nucleic acid should be present in all cells of the chicken with the potential exception of some of the haploid germ cells. The foreign nucleic acid molecule is called a "transgene" and may contain one or many genes, etc., that are not from chicken. A transgenic chicken is able to transmit the foreign nucleic acid stably in its germline.

The term "intron" refers to a sequence of DNA found in the middle of many gene sequences in most eukaryotes. These intron sequences are transcribed, but removed from within the pre-mRNA transcript before the mRNA is translated into a protein. This process of intron removal occurs by splicing together of the sequences (exons) on either side of the intron.

The term "operably-linked" refers to the association of nucleic acid sequences on a single nucleic acid fragment so that the function of one is affected by the other. For example, a promoter is operably-linked with a coding sequence when it is capable of affecting the expression of that coding sequence (i.e., the coding sequence is under the transcriptional control of the promoter). Similarly, when an intron is operably-linked to a coding sequence, the intron is spliced out of the mRNA to provide for expression of the coding sequence. In the context of gene conversion, two nucleic acids sequences are operably linked if one sequence can "donate" sequence to the other by gene conversion. If two sequences are unlinked in that one can donate sequence to the other via gene conversion, the donating sequences may be upstream or downstream of the other, and the two sequences may be proximal to each other, i.e., in that there are no other intervening genes. "Unlinked" means that the associated genetic elements are not closely associated with one another and the function of one does not affect the other.

The terms "upstream" and "downstream" are used with reference to the direction of transcription.

The term "pseudogene" is used to describe an untranscribed nucleic acid region that contains an open reading frame that may or may not contain a start and/or a stop codon. An amino acid sequence may be "encoded" by a pseudogene in the sense that the nucleotide sequence of the open reading frame can be translated in silico to produce an amino acid sequence. In the context of the heavy and light chain immunoglobulin loci, pseudogenes do not contain promoter regions, recombination signal sequences or leader sequences.

The term "homozygous" indicates that identical alleles reside at the same loci on homologous chromosomes. In contrast, "heterozygous" indicates that different alleles reside at the same loci on homologous chromosomes. A transgenic animal may be homozygous or heterozygous for a transgene.

The term "endogenous", with reference to a gene, indicates that the gene is native to a cell, i.e., the gene is present at a particular locus in the genome of a non-modified cell. An endogenous gene may be a wild type gene present at that locus in a wild type cell (as found in nature). An endogenous gene may be a modified endogenous gene if it is present at the same locus in the genome as a wild type gene. An example of such a modified endogenous gene is a gene into which a foreign nucleic acid is inserted. An endogenous gene may be present in the nuclear genome, mitochondrial genome etc.

The term "construct" refers to a recombinant nucleic acid, generally recombinant DNA, that has been generated for the purpose of the expression of a specific nucleotide sequence(s), or is to be used in the construction of other recombinant nucleotide sequences. A construct might be present in a vector or in a genome.

The term "recombinant" refers to a polynucleotide or polypeptide that does not naturally occur in a host cell. A recombinant molecule may contain two or more naturallyoccurring sequences that are linked together in a way that does not occur naturally. A recombinant cell contains a recombinant polynucleotide or polypeptide. If a cell receives a recombinant nucleic acid, the nucleic acid is "exogenous" to the cell.

The term "selectable marker" refers to a protein capable of expression in a host that allows for ease of selection of those hosts containing an introduced nucleic acid or vector. Examples of selectable markers include, but are not limited to, proteins that confer resistance to antimicrobial agents (e.g., hygromycin, bleomycin, or chloramphenicol), proteins that confer a metabolic advantage, such as a nutritional advantage on the host cell, as well as proteins that confer a functional or phenotypic advantage (e.g., cell division) on a cell.

The term "expression", as used herein, refers to the process by which a polypeptide is produced based on the nucleic acid sequence of a gene. The process includes both transcription and translation.

The term "introduced" in the context of inserting a nucleic acid sequence into a cell, means "transfection", or 'transformation" or "transduction" and includes reference to the incorporation of a nucleic acid sequence into a eukaryotic or prokaryotic cell wherein the nucleic acid sequence may be incorporated into the genome of the cell (e.g., chromosome, plasmid, plastid, or mitochondrial DNA), converted into an autonomous replicon, or transiently expressed (e.g., transfected mRNA).

The term "replacing", in the context of replacing one genetic locus with another, refers to a single step protocol or multiple step protocol.

The term "coding sequence" refers to a nucleic acid sequence encodes part of a protein, when placed under the control of appropriate regulatory elements. A coding sequence as used herein may have a continuous ORF, may be part of an ORF or might have an ORF interrupted by the presence of introns or non-coding sequences. Pseudogenes may contain an untranscribed coding sequence.

The term "in reverse orientation to" refers to coding sequences that are on different strands. For example, if a transcribed region is described as being in reverse orientation to a pseudogene, then the amino acid sequence encoded by the transcribed region is encoded by the top or bottom strand and the amino acid sequence encoded by the pseudogene is encoded by the other strand relative to the transcribed region.

The terms "antibody" and "immunoglobulin" are used interchangeably herein. These terms are well understood by those in the field, and refer to a protein consisting of one or more polypeptides that specifically binds an antigen. One form of antibody constitutes the basic structural unit of an antibody. This form is a tetramer and consists of two identical pairs of antibody chains, each pair having one light and one heavy chain. In each pair, the light and heavy chain variable regions are together responsible for binding to an antigen, and the constant regions are responsible for the antibody effector functions.

The recognized immunoglobulin polypeptides include the kappa and lambda light chains and the alpha, gamma (IgG₁, IgG₂, IgG₃, IgG₄), delta, epsilon and mu heavy chains or equivalents in other species. Full-length immunoglobulin "light chains" (of about 25 kDa or about 214 amino acids) comprise a variable region of about 110 amino acids at the NH₂-terminus and a kappa or lambda constant region at the COOH-terminus. Full-length immunoglobulin "heavy chains" (of about 50 kDa or about 446 amino acids), similarly comprise a variable region (of about 116 amino acids) and one of the aforementioned heavy chain constant regions, e.g., gamma (of about 330 amino acids).

The terms "antibodies" and "immunoglobulin" include antibodies or immunoglobulins of any isotype, fragments of antibodies which retain specific binding to antigen, including, but not limited to, Fab, Fv, scFv, and Fd fragments, chimeric antibodies, humanized antibodies, single-chain antibodies, and fusion proteins comprising an antigen-binding portion of an antibody and a non-antibody protein. The antibodies may be detectably labeled, e.g., with a radioisotope, an enzyme which generates a detectable product, a fluorescent protein, and the like. The antibodies may be further conjugated to other moieties, such as members of specific binding pairs, e.g., biotin (member of biotinavidin specific binding pair), and the like. The antibodies may also be bound to a solid support, including, but not limited to, polystyrene plates or beads, and the like. Also encompassed by the term are Fab', Fv, F(ab')₂, and or other antibody fragments that retain specific binding to antigen, and monoclonal as well as polyclonal antibodies.

Antibodies may exist in a variety of other forms including, for example, Fv, Fab, and (Fab')₂, as well as bi-functional (i.e. bi-specific) hybrid antibodies (e.g., Lanzavecchia et al., Eur. J. Immunol. 17, 105 (1987)) and in single chains (e.g., Huston et al., Proc. Natl. Acad. Sci. U.S.A., 85, 5879-5883 (1988) and Bird et al., Science, 242, 423-426 (1988). (See, generally, Hood et al., "Immunology", Benjamin, N.Y., 2nd ed. (1984), and Hunkapiller and Hood, Nature, 323, 15-16 (1986),).

An immunoglobulin light or heavy chain variable region consists of a "framework" region (FR) interrupted by three hypervariable regions, also called "complementarity determining regions" or "CDRs". The extent of the framework region and CDRs have been precisely defined (see, Lefranc et al, IMGT, the international ImMunoGeneTics information system. Nucleic Acids Res. 2009 vol. 37 (Database issue): D1006-12. Epub 2008 Oct 31; see worldwide website of imgt.org and referred to hereinafter as the "IMGT system")). The numbering of all antibody amino acid sequences discussed herein conforms to the IMGT system. The sequences of the framework regions of different light or heavy chains are relatively conserved within a species. The framework region of an antibody, that is the combined framework regions of the constituent light and heavy chains, serves to position and align the CDRs. The CDRs are primarily responsible for binding to an epitope of an antigen.

Chimeric antibodies are antibodies whose light and heavy chain genes have been constructed, typically by genetic engineering, from antibody variable and constant region genes belonging to different species. For example, the variable segments of the genes from a chicken or rabbit monoclonal antibody may be joined to human constant segments, such as gamma 1 and gamma 3. An example of a therapeutic chimeric antibody is a hybrid protein composed of the variable or antigen-binding domain from a chicken or rabbit antibody and the constant or effector domain from a human antibody (e.g., the anti-Tac chimeric antibody made by the cells of A.T.C.C. deposit Accession No. CRL 9688), although other mammalian species may be used.

As used herein, the term "human framework" refers to a framework that has an amino acid sequence that is at least 90% identical, e.g., at least 95%, at least 98% or at least 99% identical to the amino acid sequence of a human antibody, e.g., the amino acid sequence of a human germ-line sequence of an antibody. In certain cases, a human framework may be a fully human framework, in which case the framework has an amino acid sequence that is identical to that of a human antibody, e.g., a germ-line antibody.

As used herein, the term "humanized antibody" or "humanized immunoglobulin" refers to a non-human antibody containing one or more amino acids (in a framework region, a constant region or a CDR, for example) that have been substituted with a correspondingly positioned amino acid from a human antibody. In general, humanized antibodies are expected to produce a reduced immune response in a human host, as compared to a nonhumanized version of the same antibody.

It is understood that the humanized antibodies designed and produced by the present method may have additional conservative amino acid substitutions which have substantially no effect on antigen binding or other antibody functions. By conservative substitutions is intended combinations such as those from the following groups: gly, ala; val, ile, leu; asp, glu; asn, gln; ser, thr; lys, arg; and phe, tyr. Amino acids that are not present in the same group are "substantially different" amino acids.

The term "specific binding" refers to the ability of an antibody to preferentially bind to a particular analyte that is present in a homogeneous mixture of different analytes. In certain embodiments, a specific binding interaction will discriminate between desirable and undesirable analytes in a sample, in some embodiments more than about 10 to 100-fold or more (e.g., more than about 1000- or 10,000-fold).

In certain embodiments, the affinity between an antibody and analyte when they are specifically bound in an antibody/analyte complex is characterized by a K_{D} (dissociation constant) of less than 10⁻⁶ M, less than 10⁻⁷ M, less than 10⁻⁸ M, less than 10⁻⁹ M, less than 10⁻⁹ M, less than 10⁻¹¹ M, or less than about 10⁻¹² M or less.

A "variable region" of a heavy or light antibody chain is an N-terminal mature domain of the chain that contains CDR1, CDR2 and CD3, and framework regions. The heavy and light chain of an antibody both contain a variable domain. All domains, CDRs and residue numbers are assigned on the basis of sequence alignments and structural knowledge. Identification and numbering of framework and CDR residues is as defined by the IMGT system.

VH is the variable domain of an antibody heavy chain. VL is the variable domain of an antibody light chain.

As used herein the term "isolated," when used in the context of an isolated antibody, refers to an antibody of interest that is at least 60% free, at least 75% free, at least 90% free, at least 95% free, at least 98% free, and even at least 99% free from other components with which the antibody is associated with prior to purification.

The terms "treatment" "treating" and the like are used herein to refer to any treatment of any disease or condition in a mammal, e.g. particularly a human or a mouse, and includes:
a) preventing a disease, condition, or symptom of a disease or condition from occurring in a subject which may be predisposed to the disease but has not yet been diagnosed as having it;
b) inhibiting a disease, condition, or symptom of a disease or condition, e.g., arresting its development and/or delaying its onset or manifestation in the patient; and/or c) relieving a disease, condition, or symptom of a disease or condition, e.g., causing regression of the condition or disease and/or its symptoms.

The terms "subject," "host," "patient," and "individual" are used interchangeably herein to refer to any mammalian subject for whom diagnosis or therapy is desired, particularly humans. Other subjects may include cattle, dogs, cats, guinea pigs, rabbits, rats, mice, horses, and so on.

A "natural" antibody is an antibody in which the heavy and light immunoglobulins of the antibody have been naturally selected by the immune system of a multi-cellular organism, as opposed to unnaturally paired antibodies made by e.g. phage display. As such, the certain antibodies do not contain any viral (e.g., bacteriophage M13)-derived sequences. Spleen, lymph nodes and bone marrow are examples of tissues that produce natural antibodies in an animal.

The term "introduced" in the context of inserting a nucleic acid sequence into a cell, means "transfection", or 'transformation", or "transduction" and includes reference to the incorporation of a nucleic acid sequence into a eukaryotic or prokaryotic cell wherein the nucleic acid sequence may be present in the cell transiently or may be incorporated into the genome of the cell (e.g., chromosome, plasmid, plastid, or mitochondrial DNA), converted into an autonomous replicon.

The term "plurality" refers to at least 2, at least 5, at least 10, at least 20, at least 50, at least 100, at least 200, at least 500, at least 1000, at least 2000, at least 5000, or at least 10,000 or at least 50,000 or more. In certain cases, a plurality includes at least 10 to 50. In other embodiments, a plurality may be at least 50 to 1,000.

Further definitions may be elsewhere in this disclosure.

### DESCRIPTION OF EXEMPLARY EMBODIMENTS

A transgenic chicken that has a modified immunoglobulin heavy chain immunoglobulin locus is provided. As noted above and as will be described in greater detail below, the modified locus has no endogenous V-D-J region and, instead, has a human VH segment, a human D cluster, a human J segment and a plurality of upstream pseudogenes based on human V_{H} sequences. The modified IgH locus undergoes V(D)J recombination in the chicken, gene conversion between the upstream pseudogenes and the human VH segment occurs, and the chicken produces antibodies that have a diversified immunoglobulin heavy chain.

It is to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described.

It must be noted that as used herein and in the appended claims, the singular forms "a", "and", and "the" include plural referents unless the context clearly dictates otherwise. Thus, for example, reference to "a cell" includes a plurality of cells and reference to "a candidate agent" includes reference to one or more candidate agents and equivalents thereof known to those skilled in the art, and so forth. It is further noted that the claims may be drafted to exclude any optional element. As such, this statement is intended to serve as antecedent basis for use of such exclusive terminology as "solely", "only" and the like in connection with the recitation of claim elements, or use of a "negative" limitation.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

### Transgenic chickens and methods for making the same

A transgenic chicken comprising a genome comprising a modified endogenous immunoglobulin heavy chain (IgH) locus is provided. This modified locus is schematically illustrated as "SynVH-SD" in Fig. 1. As shown, the modified locus lacks the entire contiguous endogenous chicken V-D-J region. This sequence of this locus is currently unknown, but it thought to be in the range of 10-12 kb (e.g., about 11 kb) in length or at least 15 kb (e.g., 15 kb to 25 kb in length or approximately 20 kb in length). Based on the results described in the examples section below, the wild type locus does not contain any essential genes (other than the endogenous chicken V-D-J sequences), unlike other animals (e.g., mouse). Removal of the endogenous chicken V-D-J region prevents V(D)J recombination with the V-D-J human sequences described below. Such recombination events would remove much of the locus, thereby inactivating it. Removing the endogenous chicken V-D-J region prevents these events from happening.

In addition to lacking the entire contiguous endogenous chicken V-D-J region, the locus comprises in operable linkage: (i) an immunoglobulin heavy chain gene promoter, e.g., a chicken immunoglobulin heavy chain gene promoter; (ii) a germline human V_{H} segment, where the germline human V_{H} segment comprises a coding sequence for a variable domain comprising a FR1, a CDR1, a FR2, a CDR2, and a FR3. V_{H} segments are sometimes called "germline V_{H} genes", "functional V_{H} segments" or "germline V_{H} sequences" in the art. Excluding pseudogenes, there are believed to be over 40 germline V_{H} segments in the haploid human genome (see, e.g., Kohsaka et al, J. Clin. Invest. 1996 98: 2794-800) that can be grouped into at least seven families (see, e.g., Schroeder et al International Immunology, 2: 41-50 1989). In some embodiments, the human germline V_{H} segment can be from the V_{H}3 family, the V_{H}1 family or the V_{H}4 family, for example. Such segments lack heavy chain CDR3 and FR4 coding sequence.

As shown in Fig. 1, downstream of the germline human V_{H} segment, the modified locus also contains a (iii) a human D cluster (i.e., an array of, e.g., 10-27 human Diversity (D) gene segments that can be separated by sequences (i.e., intervening sequences) from the chicken genome as well as a single human Joining (J) segment (e.g., one of the 6 J segments from the human germline, see, e.g., Li et al Blood 2004 103: 4602-4409). In some embodiments, any codons for a cysteine in the D segments may have been mutated to encode another amino acid, e.g., tyrosine or tryptophan, in order to minimize disulfide bonds in the antibodies (particularly in the heavy chain CDR3 region) produced by the chicken. The recombination Signal Sequences (RSSs) flanking the variable (V), diversity (D), and joining (J) genes segments may be from chicken, but the chicken recombinase should recognize human RRSs because the sequences are almost the same.

Downstream from the J segment the locus comprises a plurality of sequences encoding constant regions that may be endogenous to the chicken. The locus contains an intron that joins the 3' end of a transcript of the J segment to the 5' end of a copy of the constant region coding sequence. As shown, the modified locus also contains a plurality of pseudogenes (e.g., at least 10 or 10 to 30 pseudogenes) upstream of the germline human V_{H} segment, where the pseudogenes are of structure FR1-CDR1-FR2-CDR2-FR3,wherein: (i) the FR1, FR2 and FR3 segments in the pseudogenes (i.e., the sequences that "encode" the heavy chain FR1, FR2 and FR3 sequences of an antibody) each have a sequence that is substantially the same as (i.e., having sequences that are at least 95% identical to or identical to) the FR1, FR2 and FR3 segments as the corresponding segments in the germline human V_{H} segment, and (ii) CDR1 and CDR2 sequences, which may encode the CDR1s and CDR2s encoded by different human V_{H} segments that are in the same family as the human germline V_{H} segment. For example, if the human germline V_{H} segment is a member of the V_{H}3 family, then the pseudogene will contain the FR1, FR2 and FR3 sequences from that segment, and CDR1/CDR2 sequences from other human germline VH segment in the V_{H}3 family. Likewise, if the human germline V_{H} segment is a member of the V_{H}1 family, then the pseudogene will contain the FR1, FR2 and FR3 sequences from that segment, and CDR1/CDR2 sequences from other human germline V_{H} segment in the V_{H}1 family, etc. In some embodiments, the pseudogenes are in reverse orientation to the germline human V_{H} segment. The germline human V_{H} segment may be the selected from the following sequences: VH1-18, VH1-2, VH1-24, VH1-3, VH1-45, VH1-46, VH1-58, VH1-69, VH1-8, VH2-26, VH2-5, VH2-70, VH3-11, VH3-13, VH3-15, VH3-16, VH3-20, VH3-21, VH3-23, VH3-30, VH3-33, VH3-35, VH3-38, VH3-43, VH3-48, VH3-49, VH3-53, VH3-64, VH3-66, VH3-7, VH3-72, VH3-73, VH3-74, VH3-9, VH4-28, VH4-31, VH4-34, VH4-39, VH4-4, VH4-59, VH4-61, VH5-51, VH6-1, and VH7-81. See PCT WO 2005/005604 for a description of the different germline sequences.

While, as noted above, the FW segments of the human germline V_{H} segment and the pseudogenes may be identical to one another, while the CDR segments of the human germline V_{H} segment and the pseudogenes may differ, thereby allowing gene conversion to occur between the CDR segments of the pseudogenes and the germline sequence. Further, the CDRs may vary in length. In certain embodiments, the heavy chain CDR1 may be in the range of 6 to 12 amino acid residues in length, the heavy chain CDR2 may be in the range of 4 to 12 amino acid residues in length, the heavy chain CDR3 may be in the range of 3 to 25 amino acid residues in length, although antibodies having CDRs of lengths outside of these ranges are envisioned.

In the configuration described above and as shown in Fig. 1, the modified IgH locus undergoes V(D)J recombination in the chicken, the plurality of germline human V_{H} pseudogenes, after V(D)J recombination, donate nucleotide sequences to the germline human V_{H} segment (to at least the CDR1 and CDR2 coding sequence in the germline human V_{H} segment) by gene conversion; and the chicken produces antibodies that comprise a diversified immunoglobulin heavy chain. The antibodies produced by the chicken are diversified in the heavy chain CDR1, CDR2 and CDR3 regions, and the median length of the heavy chain CDR3 is 11-13, e.g., about 12 residues. The CDR3 region is diversified by the D segment selected by V(D)J recombination and somatic hypermutation. The lack of TdT in chickens limits the length of the heavy chain CDR3 to what is encoded by the D genes and/or what could be inserted by gene conversion and, as such, the heavy chain CDR3 is relatively short, relative to wild type chicken and human antibodies and few D-D joins were observed.

The chicken may be homozygous for the modified IgH locus, or heterozygous for the modified IgH locus. If this chicken is or heterozygous for the modified IgH locus, the endogenous IgH locus on the homologous chromosome may be knocked out. For example, in some embodiments, the endogenous IgH locus on the homologous chromosome may lack its J region or its entire contiguous endogenous chicken V-D-J region. The chicken may also be heterozygous for a wild type IgH locus.

In certain embodiments, the transgenic chicken may also express an immunoglobulin light chain V region linked to a chicken light chain constant region or a human V region linked to a human light chain constant region, e.g., a human immunoglobulin light chain, such that the antibodies produced by the chicken are fully human or chimeric light chains with a human V region and chicken constant region.

In certain embodiments, an antibody produced by a subject transgenic animal may contain a chicken constant domain and variable domains that are human. Since an endogenous constant region may be employed in these embodiments, the antibody may still undergo class switching and affinity maturation, which allows the animal to undergo normal immune system development, and mount normal immune responses. In specific embodiments transgenic chickens have three endogenous constant regions in the heavy chain locus encoding IgM, IgY and IgA. During the early stages of B cell development, B cells express IgM. As affinity maturation proceeds, class switching converts the constant region into IgY or IgA. IgY provides humoral immunity to both adults and neonatal chicks which receive about 200 mg of IgY via a reserve deposited into egg yolk. IgA is found primarily in lymphoid tissues (e.g., the spleen, Peyer's patches and Harderian glands) and in the oviduct although a small amount is transferred into eggs and subsequently taken up by the developing embryo.

A transgenic chicken lacking its entire contiguous endogenous chicken V-D-J region in one or both of its homologous chromosomes, as described above, is also provided. A B cell derived from the transgenic chicken is also provided.

A method of making the transgenic chicken is provided. In certain embodiments, the method comprises: (a) removing the entire contiguous endogenous chicken V-D-J region from the immunoglobulin heavy chain (IgH) locus of a chicken; and (b) inserting into the locus a construct comprising: (i) an immunoglobulin heavy chain gene promoter; (ii) a germline human V_{H} segment comprising a coding sequence for a variable domain comprising a FR1, a CDR1, a FR2, a CDR2, and a FR3; (iii) a human D cluster; (iv) a human J segment; and (vi) a plurality of pseudogenes that each comprise: substantially the same FR1, FR2 and FR3 coding sequences as the functional human V_{H} segment of (b)(ii); and CDR1 and CDR2 coding sequences. Steps (a) and (b) can be done in any order. However, non-coding sequences (introns) may be retained in endogenous configuration in order to preserve endogenous regulatory elements that may be contained within.

Once a subject transgenic animal is made, antibodies against an antigen can be readily obtained by immunizing the animal with the antigen. A variety of antigens can be used to immunize a transgenic host animal. Such antigens include, microorganism, e.g. viruses and unicellular organisms (such as bacteria and fungi), alive, attenuated or dead, fragments of the microorganisms, or antigenic molecules isolated from the microorganisms.

In some embodiments, this method may comprise: (a) immunizing a transgenic chicken as described above with an antigen; and (b) obtaining from the transgenic chicken an antibody, e.g., a polyclonal or monoclonal antibody, that specifically binds to the antigen. Also described is where the method could comprise (c) making hybridomas using B cells of the transgenic chicken; and (d) screening said hybridomas to identify a hybridoma that produces an antibody that specifically binds to the antigen. Further is described where the method could further comprise using PCR to amplify the heavy and light chain variable region-encoding nucleic acid from B cells of the transgenic animal, and expressing a recombinant antibody using said amplified nucleic acid. Monoclonal antibodies may also be recovered from the transgenic chickens using the GEM assay (US patents 8030095 and 8415173; Izquierdo et al, 2014), by deep sequencing or any other B cell interrogation technology (see, e.g., Abcellera's website).

In certain embodiments, the transgenic chicken may be immunized with: GD2, EGF-R, CEA, CD52, CD20, Lym-1, CD6, complement activating receptor (CAR), EGP40, VEGF, tumor-associated glycoprotein TAG-72 AFP (alpha-fetoprotein), BLyS (TNF and APOL - related ligand), CA125 (carcinoma antigen 125), CEA (carcinoembrionic antigen), CD2 (T-cell surface antigen), CD3 (heteromultimer associated with the TCR), CD4, CD11a (integrin alpha-L), CD14 (monocyte differentiation antigen), CD20, CD22 (B-cell receptor), CD23 (low affinity IgE receptor), CD25 (IL-2 receptor alpha chain), CD30 (cytokine receptor), CD33 (myeloid cell surface antigen), CD40 (tumor necrosis factor receptor), CD44v6 (mediates adhesion of leukocytes), CD52 (CAMPATH-1), CD80 (costimulator for CD28 and CTLA-4), complement component C5, CTLA, EGFR, eotaxin (cytokine A11), HER2/neu, HER3, HLA-DR, HLA-DR10, HLA ClassII, IgE, GPiib/iiia (integrin), Integrin aVβ3, Integrins a4β1 and a4β7, Integrin β2, IFN-gamma, IL-1β, IL-4, IL-5, IL-6R (IL6 receptor), IL-12, IL-15, KDR (VEGFR-2), lewisy, mesothelin, MUC1, MUC18, NCAM (neural cell adhesion molecule), oncofetal fibronectin, PDGFβR (Beta platelet-derived growth factor receptor), PMSA, renal carcinoma antigen G250, RSV, E-Selectin, TGFbeta1, TGFbeta2, TNF**α**, DR4, DR5, DR6, VAP-1 (vascular adhesion protein 1) or VEGF, or the like in order to produce a therapeutic antibody.

The antigens can be administered to a transgenic chicken in any convenient manner, with or without an adjuvant, and can be administered in accordance with a predetermined schedule.

After immunization, serum from the immunized transgenic animals can be fractionated for the purification of pharmaceutical grade polyclonal antibodies specific for the antigen. In the case of transgenic birds, antibodies can also be made by fractionating egg yolks. A concentrated, purified immunoglobulin fraction may be obtained by chromatography (affinity, ionic exchange, gel filtration, etc.), selective precipitation with salts such as ammonium sulfate, organic solvents such as ethanol, or polymers such as polyethyleneglycol.

For making a monoclonal antibody, antibody-producing cells, e.g., spleen cells or other cells, may isolated from the immunized transgenic animal and used either in cell fusion with transformed cell lines for the production of hybridomas, or cDNAs encoding antibodies are cloned by standard molecular biology techniques and expressed in transfected cells. The procedures for making monoclonal antibodies are well established in the art. See, e.g., European Patent Application 0 583 980 A1, U.S. Pat. No. 4,977,081, WO 97/16537, and EP 0 491 057 B1. In vitro production of monoclonal antibodies from cloned cDNA molecules has been described by Andris-Widhopf et al., , J Immunol Methods 242:159 (2000), and by Burton, Immunotechnology 1:87 (1995).

### Antibody compositions and method of screening

Antibody compositions are provided, but are not part of the invention. As noted above, the heavy and light chains variable domains of the antibody are naturally paired by the immune system of the animal. Such antibodies may, in certain case, be post-translationally modified (e.g., glycosylated) by the host cell and may have a glycosylation pattern and composition characteristic of the species of transgenic chicken.

The antibodies produced by the subject transgenic chicken may be screened to identify an antibody of interest. In general, this method involves producing a plurality of hybrid cells producing monoclonal antibodies using the method described above, and screening the plurality of monoclonal antibodies using one or a combination of a variety of assays. In general, these assays are functional assays, and may be grouped as follows: assays that detect an antibody's binding affinity or specificity, and assays that detect the ability of an antibody to inhibit a process.

A monoclonal antibody identified as having a specific binding activity with an antigen, or an inhibitory activity is termed a monoclonal antibody of interest.

### Binding assays

In these assays, antibodies are tested for their ability to bind specifically to a substrate. The term "specifically" in the context of antibody binding, refers to high avidity and/or high affinity binding of an antibody to a specific antigen i.e., a polypeptide, or epitope. In many embodiments, the specific antigen is an antigen (or a fragment or subfraction of an antigen) used to immunize the animal host from which the antibody-producing cells were isolated. Antibody specifically binding an antigen or fragment thereof is stronger than binding of the same antibody to other antigens. Antibodies which bind specifically to a polypeptide may be capable of binding other polypeptides at a weak, yet detectable, level (e.g., 10% or less of the binding shown to the polypeptide of interest). Such weak binding, or background binding, is readily discernible from the specific antibody binding to a subject polypeptide, e.g. by use of appropriate controls. In general, specific antibodies bind to an antigen with a binding affinity of 10⁻⁷ M or more, e.g., 10⁻⁸ M or more (e.g., 10⁻⁹ M, 10⁻¹⁰, 10⁻¹¹, etc.). In general, an antibody with a binding affinity of 10⁻⁶ M or less is not useful in that it will not bind an antigen at a detectable level using conventional methodology currently used.

Typically, in performing a screening assay, antibody samples produced by a library of antibody producing host cells are deposited onto a solid support in a way that each antibody can be identified, e.g. with a plate number and position on the plate, or another identifier that will allow the identification of the host cell culture that produced the antibody.

The antibodies disclosed herein may be screened for immunospecific binding by any method known in the art. The immunoassays which can be used include but are not limited to competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, and protein A immunoassays, to name but a few. Such assays are routine and well known in the art (see, e.g., Ausubel et al, eds, 1994, Current Protocols in Molecular Biology, Vol. 1, John Wiley & Sons, Inc., New York). Exemplary immunoassays are described briefly below (but are not intended by way of limitation).

Immunoprecipitation protocols generally involve lysing a population of cells in a lysis buffer such as RIPA buffer (1% NP-40 or Triton X-100, 1% sodium deoxycholate, 0.1% SDS, 0.15 M NaCl, 0.01 M sodium phosphate at pH 7.2, 1% Trasylol) supplemented with protein phosphatase and/or protease inhibitors (e.g., EDTA, PMSF, aprotinin, sodium vanadate), adding the antibody of interest to the cell lysate, incubating for a period of time (e.g., 1-4 hours) at 4.degree. C., adding protein A and/or protein G sepharose beads to the cell lysate, incubating for about an hour or more at 4°C., washing the beads in lysis buffer and resuspending the beads in SDS/sample buffer. The ability of the antibody of interest to immunoprecipitate a particular antigen can be assessed by, e.g., western blot analysis. One of skill in the art would be knowledgeable as to the parameters that can be modified to increase the binding of the antibody to an antigen and decrease the background (e.g., preclearing the cell lysate with sepharose beads).

Western blot analysis generally involves preparation of protein samples followed by electrophoresis of the protein samples in a polyacrylamide gel (e.g., 8%-20% SDS-PAGE depending on the molecular weight of the antigen), and transfer of the separated protein samples from the polyacrylamide gel to a membrane such as nitrocellulose, PVDF or nylon. Following transfer, the membrane is blocked in blocking solution (e.g., PBS with 3% BSA or non-fat milk), washed in washing buffer (e.g., PBS-Tween 20), and incubated with primary antibody (the antibody of interest) diluted in blocking buffer. After this incubation, the membrane is washed in washing buffer, incubated with a secondary antibody (which recognizes the primary antibody, e.g., an anti-human antibody) conjugated to an enzymatic substrate (e.g., horseradish peroxidase or alkaline phosphatase) or radioactive molecule (e.g., 32P or 1251), and after a further wash, the presence of the antigen may be detected. One of skill in the art would be knowledgeable as to the parameters that can be modified to increase the signal detected and to reduce the background noise.

ELISAs involve preparing antigen, coating the well of a 96 well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art.

The binding affinity of an antibody to an antigen and the off-rate of an antibodyantigen interaction can be determined by competitive binding assays. One example of a competitive binding assay is a radioimmunoassay comprising the incubation of labeled antigen (e.g., 3H or 1251) with the antibody of interest in the presence of increasing amounts of unlabeled antigen, and the detection of the antibody bound to the labeled antigen. The affinity of the antibody of interest for a particular antigen and the binding off-rates can be determined from the data by scatchard plot analysis. Competition with a second antibody can also be determined using radioimmunoassays. In this case, the antigen is incubated with antibody of interest conjugated to a labeled compound (e.g., 3H or 125I) in the presence of increasing amounts of an unlabeled second antibody.

Antibodies disclosed herein may be screened using immunocytochemisty methods on cells (e.g., mammalian cells, such as CHO cells) transfected with a vector enabling the expression of an antigen or with vector alone using techniques commonly known in the art. Antibodies that bind antigen transfected cells, but not vector-only transfected cells, are antigen specific.

In certain embodiments, however, the assay is an antigen capture assay, and an array or microarray of antibodies may be employed for this purpose. Methods for making and using microarrays of polypeptides are known in the art (see e.g. U.S. patents 6,372,483, 6,352,842, 6,346,416 and 6,242,266).

### Inhibitor assays

In certain embodiments, the assay measures the specific inhibition of an antibody to an interaction between a first compound and a second compound (e.g. two biopolymeric compounds) or specifically inhibits a reaction (e.g. an enzymatic reaction). In the interaction inhibition assay, one interaction substrate, usually a biopolymeric compound such as a protein e.g. a receptor, may be bound to a solid support in a reaction vessel. Antibody is added to the reaction vessel followed by a detectable binding partner for the substrate, usually a biopolymeric compound such as a protein e.g. a radiolabeled ligand for the receptor. After washing the vessel, interaction inhibition may be measured by determining the amount of detectable binding partner present in the vessel. Interaction inhibition occurs when binding of the binding partner is reduced greater than about 20%, greater than about 50%, greater than about 70%, greater than about 80%, or greater than about 90% or 95% or more, as compared to a control assay that does not contain antibody.

In the reaction inhibition assay, an enzyme may be bound to a solid support in a reaction vessel. Antibody is usually added to the reaction vessel followed by a substrate for the enzyme. In many embodiments, the products of the reaction between the enzyme and the substrate are detectable, and, after a certain time, the reaction is usually stopped. After the reaction has been stopped, reaction inhibition may be measured by determining the level of detectable reaction product present in the vessel. Reaction inhibition occurs when the rate of the reaction is reduced greater than about 20%, greater than about 50%, greater than about 70%, greater than about 80%, or greater than about 90% or 95% or more, as compared to a control assay that does not contain antibody.

### In vivo assays

In certain embodiments the monoclonal antibodies are tested in vivo. In general, the method involves administering a subject monoclonal antibody to an animal model for a disease or condition and determining the effect of the monoclonal antibody on the disease or condition of the model animal. *In vivo* assays disclosed herein include controls, where suitable controls include a sample in the absence of the monoclonal antibody. Generally a plurality of assay mixtures is run in parallel with different antibody concentrations to obtain a differential response to the various concentrations. Typically, one of these concentrations serves as a negative control, i.e. at zero concentration or below the level of detection.

A monoclonal antibody of interest is one that modulates, i.e., reduces or increases a symptom of the animal model disease or condition by at least about 10%, at least about 20%, at least about 25%, at least about 30%, at least about 35%, at least about 40%, at least about 45%, at least about 50%, at least about 55%, at least about 60%, at least about 65%, at least about 70%, at least about 80%, at least about 90%, or more, when compared to a control in the absence of the antibody. In general, a monoclonal antibody of interest will cause a subject animal to be more similar to an equivalent animal that is not suffering from the disease or condition. Monoclonal antibodies that have therapeutic value that have been identified using the methods and compositions disclosed herein are termed "therapeutic" antibodies.

Since a hybrid cell expressing an antibody of interest contains immunoglobulin heavy and light chain-encoding nucleic acids, the nucleic acids encoding the monoclonal antibody of interest may be identified if the host cell expressing the monoclonal antibody of interest is identified. As such, the subject nucleic acids may be identified by a variety of methods known to one of skill in the art. Similar methods are used to identify host cell cultures in monoclonal antibody production using hybridoma technology (Harlow et al., Antibodies: A Laboratory Manual, First Edition (1988) Cold spring Harbor, N.Y.).

For example, upon identifying a monoclonal antibody of interest, the host cell expressing the antibody of interest may be identified using a "look-up" table which lists, for every antibody sample, the corresponding host cell culture. In certain other embodiments, a look-up table containing antibody library sample identifiers, corresponding expression cassette library sample identifiers and/or host cell identifiers may be used to identify the subject nucleic acids.

Once identified, the nucleic acids encoding a monoclonal antibody of interest may be recovered, characterized and manipulated using techniques familiar to one of skill in the art (Ausubel, et al, Short Protocols in Molecular Biology, 3rd ed., Wiley & Sons, (1995) and Sambrook, et al, Molecular Cloning: A Laboratory Manual, Third Edition, (2001) Cold Spring Harbor, N.Y.).

### Antibody expression

Also described are several methods of producing a monoclonal antibody of interest. In general these methods involve incubating a host cell containing a nucleic acid encoding a monoclonal antibody of interest under conditions sufficient for production of the antibody.

In some embodiments, the methods of producing a monoclonal antibody of interest involve transferring identified expression cassettes for a monoclonal antibody of interest into a suitable vector, and transferring the recombinant vector into a host cell to provide for expression of the monoclonal antibody. In some embodiments, the subject methods involve transferring at least the variable domain-encoding sequences from the identified heavy and light chains into vectors suitable for their expression in immunoglobulin heavy and light chains. Suitable constant domain-encoding sequences and/or other antibody domain-encoding sequences may be added to the variable domain-encoding sequences at this point. These nucleic acid modifications may also allow for humanization of the subject antibody.

The subject monoclonal antibodies can be produced by any method known in the art for the synthesis of antibodies, in particular, by recombinant expression techniques.

Recombinant expression of a subject monoclonal antibody, or fragment, derivative or analog thereof, usually requires construction of an expression vector containing a polynucleotide that encodes the antibody. Methods which are well known to those skilled in the art can be used to construct expression vectors containing antibody coding sequences and appropriate transcriptional and translational control signals. These methods include, for example, in vitro recombinant DNA techniques and synthetic techniques. As such, disclosed herein are vectors comprising a nucleotide sequence encoding an antibody molecule

The expression vector is transferred to a host cell by conventional techniques and the transfected cells are then cultured to produce a subject antibody. In most embodiments, vectors encoding both the heavy and light chains are co-expressed in the host cell to provide for expression of the entire immunoglobulin molecule.

A variety of host-expression vector systems may be utilized to express a subject monoclonal antibody. These include but are not limited to microorganisms such as bacteria (e.g., E. coli, B. subtilis) transformed with recombinant bacteriophage DNA, plasmid DNA or cosmid DNA expression vectors containing antibody coding sequences; yeast (e.g., Saccharomyces, Pichia) transformed with recombinant yeast expression vectors containing antibody coding sequences; insect cell systems infected with recombinant virus expression vectors (e.g., baculovirus) containing antibody coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing antibody coding sequences; or mammalian cell systems (e.g., COS, CHO, BHK, 293, 3T3 cells etc.) harboring recombinant expression constructs containing promoters derived from the genome of mammalian cells (e.g., metallothionein promoter) or from mammalian viruses (e.g., the adenovirus late promoter; the vaccinia virus 7.5K promoter). In many embodiments, bacterial cells such as Escherichia coli, and eukaryotic cells are used for the expression of entire recombinant antibody molecules. For example, mammalian cells such as Chinese hamster ovary cells (CHO), in conjunction with a vector such as the major intermediate early gene promoter element from human cytomegalovirus is an effective expression system for antibodies (Foecking et al., Gene 45:101 (1986); Cockett et al., Bio/Technology 8:2 (1990)).

In bacterial systems, a number of expression vectors may be selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited, to the E. coli expression vector pUR278 (Ruther et al., EMBO J. 2:1791 (1983)), in which the antibody coding sequence may be ligated individually into the vector in frame with the lac Z coding region so that a fusion protein is produced; pIN vectors (Inouye & Inouye, Nucleic Acids Res. 13:3101-3109 (1985); Van Heeke & Schuster, J. Biol. Chem. 24:5503-5509 (1989)); and the like. pGEX vectors may also be used to express foreign polypeptides as fusion proteins with glutathione S-transferase (GST). In general, such fusion proteins are soluble and can easily be purified from lysed cells by adsorption and binding to matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa califomica* nuclear polyhedrosis virus (AcNPV) is used as a vector to express antibodies. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized to express a subject antibody. In cases where an adenovirus is used as an expression vector, the antibody coding sequence of interest may be ligated to an adenovirus transcription/translation control complex, e.g., the late promoter and tripartite leader sequence. This chimeric gene may then be inserted in the adenovirus genome by in vitro or in vivo recombination. Insertion in a non-essential region of the viral genome (e.g., region E1 or E3) will result in a recombinant virus that is viable and capable of expressing the antibody molecule in infected hosts. (e.g., see Logan & Shenk, Proc. Natl. Acad. Sci. USA 81:355-359 (1984)). The efficiency of expression may be enhanced by the inclusion of appropriate transcription enhancer elements, transcription terminators, etc. (see Bittner et al., Methods in Enzymol. 153:51-544 (1987)).

For long-term, high-yield production of recombinant antibodies, stable expression may be used. For example, cell lines, which stably express the antibody molecule may be engineered. Rather than using expression vectors which contain viral origins of replication, host cells can be transformed with immunoglobulin expression cassettes and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into a chromosome and grow to form foci which in turn can be cloned and expanded into cell lines. Such engineered cell lines may be particularly useful in screening and evaluation of compounds that interact directly or indirectly with the antibody molecule.

A number of selection systems may be used, including but not limited to the herpes simplex virus thymidine kinase (Wigler et al., Cell 11:223 (1977)), hypoxanthine-guanine phosphoribosyltransferase (Szybalska & Szybalski, Proc. Natl. Acad. Sci. USA 48:202 (1992)), and adenine phosphoribosyltransferase (Lowy et al., Cell 22:817 (1980)) genes can be employed in tk-, hgprt- or aprt-cells, respectively. Also, antimetabolite resistance can be used as the basis of selection for the following genes: dhfr, which confers resistance to methotrexate (Wigler et al., Natl. Acad. Sci. USA 77:357 (1980); O'Hare et al., Proc. Natl. Acad. Sci. USA 78:1527 (1981)); gpt, which confers resistance to mycophenolic acid (Mulligan & Berg, Proc. Natl. Acad. Sci. USA 78:2072 (1981)); neo, which confers resistance to the aminoglycoside G-418 Clinical Pharmacy 12:488-505; Wu and Wu, Biotherapy 3:87-95 (1991); Tolstoshev, Ann. Rev. Pharnacol. Toxicol. 32:573-596 (1993); Mulligan, Science 260:926-932 (1993); and Morgan and Anderson, Ann. Rev. Biochem. 62:191-217 (1993); TIB TECH 11(5):155-215 (1993)); and hygro, which confers resistance to hygromycin (Santerre et al., Gene 30:147 (1984)). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., J. Mol. Biol. 150:1 (1981).

The host cell may be co-transfected with two expression vectors, the first vector encoding a heavy chain derived polypeptide and the second vector encoding a light chain derived polypeptide. The two vectors may contain different selectable markers and origins of replication, which enable equal expression of heavy and light chain polypeptides. Alternatively, a single vector may be used which encodes, and is capable of expressing, both heavy and light chain polypeptides.

Once an antibody molecule has been produced, it may be purified by any method known in the art for purification of an immunoglobulin molecule, for example, by chromatography (e.g., ion exchange, affinity, particularly by affinity for the specific antigen after Protein A, and sizing column chromatography), centrifugation, differential solubility, or by any other standard technique for the purification of proteins. In many embodiments, antibodies are secreted from the cell into culture medium and harvested from the culture medium.

### Utility

Also described is a method for modulating or treating at least one antigen-related disease, in a cell, tissue, organ, animal, or patient, as known in the art or as described herein, using at least one antibody, e.g., administering or contacting the cell, tissue, organ, animal, or patient with a therapeutic effective amount of antibody. Disclosed herein, but not part of the invention, is a method for modulating or treating at least one antigen related disease, in a cell, tissue, organ, animal, or patient including, but not limited to, at least one of obesity, an immune related disease, a cardiovascular disease, an infectious disease, a malignant disease or a neurologic disease.

Typically, treatment of pathologic conditions is effected by administering an effective amount or dosage of at least one antibody composition that total, on average, a range from at least about 0.01 to 500 milligrams of at least one antibody per kilogram of patient per dose, and, preferably, from at least about 0.1 to 100 milligrams antibody/kilogram of patient per single or multiple administration, depending upon the specific activity of the active agent contained in the composition. Alternatively, the effective serum concentration can comprise 0.1-5000 ng/ml serum concentration per single or multiple administration. Suitable dosages are known to medical practitioners and will, of course, depend upon the particular disease state, specific activity of the composition being administered, and the particular patient undergoing treatment. In some instances, to achieve the desired therapeutic amount, it can be necessary to provide for repeated administration, i.e., repeated individual administrations of a particular monitored or metered dose, where the individual administrations are repeated until the desired daily dose or effect is achieved.

A subject antibody can, in certain embodiments also be used in diagnostics where the antibody is conjugated to a detectable markers or used as primary antibodies with secondary antibodies that are conjugated to detectable markers. Detectable markers, include radioactive and non-radioactive labels and are well-known to those with skill in the art. Common non-radioactive labels include detectable enzymes such as horseradish peroxidase, alkaline phosphatase and fluorescent molecules. Fluorescent molecules absorb light at one wavelength and emit it at another, thus allowing visualization with, e.g., a fluorescent microscope. Spectrophotometers, fluorescence microscopes, fluorescent plate readers and flow sorters are well-known and are often used to detect specific molecules which have been made fluorescent by coupling them covalently to a fluorescent dye. Fluorochromes such as green fluorescent protein, red shifted mutants of green fluorescent protein, amino coumarin acetic acid (AMCA), fluorescein isothiocyanate (FITC), tetramethylchodamine isothiocyanate (TRITC), Texas Red, Cy3.0 and Cy5.0 are examples of useful labels.

The molecules can be used in cell isolation strategies such as fluorescence-activated cell sorting (FACS) if fluorescent markers are used. In fluorescence-activated cell sorting, cells tagged with fluorescent molecules are sorted electronically on a flow cytometer such as a Becton-Dickinson (San Jose, Calif.) FACS IV cytometer or equivalent instrument. The fluorescent molecules are antibodies that recognize specific cell surface antigens. The antibodies are conjugated to fluorescent markers such as fluorescein isothiocyanate (FITC) or Phycoerythrin (PE).

### EXAMPLES

The following examples are provided in order to demonstrate and further illustrate certain embodiments and aspects of the present invention and are not to be construed as limiting the scope thereof.

### EXAMPLE1

### CREATION OF SYNVH-SD CHICKENS

In this study, transgenic chickens were engineered to produce a human variable region antibody repertoire. From a pharmaceutical standpoint, the use of a single V framework in chickens has an advantage because a preferred framework with optimal manufacturing and developability characteristics may be selected. Transgenes can be designed such that diversity is focused in the CDRs, while maintaining germline or neargermline framework sequences. The strategy is to retain the advantage of the chicken B cell system for incorporating diversity, using a single human framework with upstream human-based pseudogenes, instead of inserting a large human genomic fragment (carrying multiple human V, D and J genes) which may not be regulated properly in the chicken. For the single human framework to provide therapeutic candidates to any potential target, the level of diversity produced by the transgene must be sufficient. Two lines of transgenic chickens were produced: one carrying a pre-rearranged, functional VH region that relies entirely on gene conversion/somatic hypermutation to produce diversity, and one which undergoes V(D)J rearrangement first to produce a functional VH region, thereby potentially increasing the level of CDR-H3 diversity, followed by gene conversion/somatic hypermutation.

### Materials and Methods

The pre-rearranged VH region construct, SynVH-C, was previously described (18). The V(D)J rearranging construct, SynVH-SD, was made by gene synthesis of several parts followed by ligation. The V, D and J regions were assembled as follows. The human germline VH3-23*01 gene was the single V gene used, and the JH6 gene was the single J gene. 24 non-redundant human Ds were flanked by recombination signal sequences and intervening regions from the chicken D locus (these spacers were about 100-200 bp each). Cysteine codons in the human D2 family were mutated to encode tyrosine (7 instances) or tryptophan (2 instances). The rearranging elements were cloned with the chicken VH promoter to drive expression of the heavy chain, and a short section of the chicken J-C intron for splicing to the endogenous constant regions was included. The human pseudogenes contained FRs from the human germline VH3-23 gene and CDRs 1 and 2 from the VH3 germline gene family. 13 pseudogenes were designed. Spacer sequences between each pseudogene were from the chicken pseudogene region, but did not include chicken V sequences themselves. An attB site for insertion into the attP site targeted to the chicken heavy chain locus was included (21,22), and a loxP site for later recombination with loxP sites in the target genome. The SynVH-SD construct was transfected into the heavy chain attP-containing cells. In these cells, a loxP site had previously been inserted by CRISPR-mediated targeting upstream of the chicken VH segment (22). After insertion of the SynVH-SD transgene, a second loxP site was brought in by the SynVH-SD transgene, in the same orientation as the first loxP site, directly upstream of the human pseudogene array. Breeding to Cre hens removed all of the DNA between the loxP sites, which included the chicken VH and D genes and the selectable markers used during the transfections, leading to the structure shown in Fig. 1. For the light chain, all of the transgenic chickens used in this study expressed a human V-kappa light chain, from construct SynVK-CK (18). The light and heavy chains in these birds consisted of human variable regions and chicken constant regions. At the heavy chain locus, the transgenes were heterozygous in all cases, with the knockout on the other allele, giving the genotype *IgH^{SynVH}*/*IgH^{JH-KO}.* At the light chain locus, the genotype was always *IgL^{SynVK-CK}*/*IgL^{VJC-KO}.*

Chickens containing SynVH-C were immunized as described (18). SynVH-SD birds were immunized either with PGRN protein, or with a combination of DNA and PGRN protein, on a schedule similar to the SynVH-C birds.

Spleen lymphocytes were prepared by Ficoll density centrifugation. Total RNA from spleen lymphocytes (approximately 10⁷) was extracted using RNeasy (Qiagen). 10-15 ng total RNA was used in a OneStep Ahead (Qiagen) proofreading polymerase reaction using primers chVH-F9 (5'-CACCAGTCGGCTCCGCAACCATG-3' (SEQ ID NO: 1)) and cIgY-NGS-R (5'-GGGCGATGTGGGGCTCGC-3' (SEQ ID NO: 2)). Amplicons of approximately 450 bp were obtained and sequenced at ABM (Richmond, BC, Canada). ABM performed paired end merging, cluster analysis, matching of sequences to the previously identified mAb sequences, and CDR-H3 length determination.

Sequences were aligned and analyzed using DNAstar software. Excel macros were downloaded from the web site of Annemarie Honegger (University of Zurich) and used for calculating amino acid frequencies.

Animal experiments were done in accordance to Ligand Pharmaceuticals IACUC approved protocols and under supervision of the IACUC committee.

### Results

Human VH sequences from chickens with two different human heavy chain transgenes were analyzed. One of these transgenes, SynVH-C, contained a pre-rearranged functional V region (18), and the other, SynVH-SD, contained germline V, D and J segments that undergo rearrangement in B cells (Fig 1). The V region in SynVH-C was obtained from screening a human library and it contained a rearranged VH3-23/D1/JH4 region, with 9 framework (FR) changes relative to the germline VH3-23 gene. The SynVH-SD construct contained a single germline V gene, VH3-23, all of the human D elements, and a single JH6 gene. The D elements were separated by intervening sequences from chicken D locus, including the highly conserved recombination signal sequences. All of the other non-coding sequence (promoter and introns) in both constructs was derived from the chicken heavy chain locus for optimal transcriptional and post-transcriptional regulation. The transgene constructs were inserted into the endogenous heavy chain locus via a combination of gene targeting and integrase-mediated insertion (18,22). The human V regions in both transgenes splice to the endogenous downstream chicken constant regions.

Both of the transgenes contained upstream human-based pseudogenes which can participate in gene conversion of the functional human V ((8,11). The pseudogenes were designed with diversity mainly in the CDRs (FIG. 8), although some of the SynVH-C pseudogenes also contain changes in the frameworks. These pseudogenes were designed *de novo* and bear no relation to the V gene pseudogenes resident in the human genome. Two different approaches to pseudogene CDR design were taken. In SynVH-C, CDRs were derived from naturally occurring CDR sequences found in human sequence databases of expressed sequences (ESTs). All three CDRs were included in the pseudogenes, and the 3' end of each pseudogene includes CDR3 but does not extend into FR4 or include the invariant Trp-118 residue marking the border of CDR3. Between CDR3 of one pseudogene and the beginning (FR1) of the next pseudogene, spacer sequences of 100 bp were placed. In SynVH-SD, the CDRs were from the germline human VH3 family members, CDR1 and 2, with no specific CDR3 sequences in the pseudogenes, since germline V genes do not contain CDR3. Downstream of FR3 were placed the spacer sequences, which are diverse sequences that have the potential to be used in gene conversion even though they are not derived from human CDR3s (Fig. 8). After insertion, the chicken pseudogene array was still present upstream in both cases. In the case of SynVH-C, the single chicken germline VH and D cluster that normally undergo VDJ rearrangement was still present, but they should not be able to recombine into a functional V region because there is no germline JH region, and the human V region downstream is fully rearranged. In the SynVH-SD transgene, the chicken V and D genes were deleted (22) to eliminate the possibility that the chicken genes would directly recombine with the human JH gene and displace the human V and D genes. The only JH region present in the genome for both lines of birds was the human JH in the SynVH transgene (Fig 1).

Heavy chain V regions were sequenced in bulk by NGS amplicon sequencing from spleen lymphocyte populations of 9 immunized birds (see below for details of immunization). The VH regions were amplified from lymphocyte RNA following reverse transcription, using a forward primer in the 5' UTR and a reverse primer in the IgY constant region CH1 domain, and sequenced by MiSeq (Applied Biological Materials, Canada). The primers used to amplify VH regions were chosen so that they would amplify the human V region or the chicken V region, should it be expressed. In the human transgenes, the only sequence that is human is the V region coding sequence. All of the noncoding sequences (the 5' UTR, introns, etc.) and the constant region coding sequences were chicken sequence in both SynVH constructs. Paired-end reads were assembled and translated to provide theoretical protein sequence. The number of reads, unique nucleotide and protein sequences from each bird are given in Table 1. The most common sequence in each bird ranged in the number of times it was found from about 6700 to 89,000. For some of the analysis, the top 1000 most common unique sequences from each sample were used, which represented 34-62% of the total sequence data from each sample.

**Table 1. Summary of sequencing data.**

| Samples from 6 SynVH-C and 3 SynVH-SD birds are listed by their bird number. The numbers of unique nucleotide and protein sequences are given, and the number of unique peptide sequences that were sequenced two or more times (2X depth). The proportion of the total sequence reads represented by the top 1000 sequences is given in the last column. | | | | | | |
|---|---|---|---|---|---|---|
| Sample | % merged by FLASH | # unique nucleotide seqs | # unique peptide seqs | # unique pep at 2x depth | # hits of top sequence | Top 1K % of total seq |
| SynVH-C | | | | | | |
| 23806 | 90.56 | 676328 | 434570 | 41751 | 41745 | 41.27% |
| 23824 | 90.35 | 940993 | 632148 | 81377 | 28173 | 40.06% |
| 24317 | 92.17 | 930254 | 637959 | 60330 | 17049 | 53.55% |
| 26934 | 91.00 | 791431 | 540981 | 70701 | 20487 | 40.62% |
| 27022 | 88.80 | 925088 | 582374 | 92873 | 20228 | 41.81% |
| 27023 | 90.66 | 1006989 | 636318 | 68348 | 26221 | 62.30% |

| SynVH-SD | | | | | | |
|---|---|---|---|---|---|---|
| 29406 | 90.27 | 387390 | 254102 | 34733 | 6706 | 36.00% |
| 29407 | 90.18 | 696921 | 409293 | 56921 | 89009 | 46.85% |
| 29409 | 89.43 | 939220 | 571584 | 76879 | 17450 | 34.01% |

### V region and signal peptide usage

Analysis of VH regions from the 9 birds started with determining whether the expressed sequences were fully human, as expected, or contained any chicken sequence (Table 2). All of the V regions from SynVH-SD were human sequence, which was expected since the endogenous chicken V region was deleted upstream of the SynVH-SD insertion. Unexpectedly, about 5% of the sequences from the SynVH-C transgene were chicken VH regions. These sequences were comprised of chicken CDRs 1-2 and FRs 1-3, fused to CDR3 and the human J sequence, which is the only possibility since the only JH sequence present in the genome of the transgenic chickens is the human J on the SynVH transgene. CDR-H3 appeared to be human, since there are no non-canonical cysteines, which would be expected in chicken CDR-H3s (17). There were two potential sources of chicken VH sequences in the expressed antibody repertoire in SynVH: gene conversion from the chicken pseudogenes, which are still present upstream of the human pseudogenes, or gene replacement of the human functional V with the chicken functional V by a secondary rearrangement type mechanism in which the chicken V gene rearranged with the human V gene via a cryptic recombination signal sequence at the 3' end of the V gene (23-26). It seems most likely that these chicken V regions came from secondary rearrangement, fusing in frame at the human FR3-CDR3 junction in the transgene, thereby deleting the human V gene. The main evidence to favor gene replacement over gene conversion is that the signal peptide sequence was also chicken, whereas the pseudogenes do not contain signal peptide sequences and thus could not mutate the human signal peptide to a chicken signal peptide sequence. In addition, if gene conversion could replace the expressed V region with the chicken V region, then one might expect the same occurrence in the SynVH-SD sequences, which also contains the upstream pseudogenes. However, no V replacement was observed in SynVH-SD. The functional chicken V and D cluster have been deleted upstream of the SynVH-SD transgene which precludes the possibility of gene replacement.

The signal peptide did not always match the sequence that was introduced in the transgene construct. Although 80-90% of the sequences in SynVH-C and SynVH-SD transgenes contained the human signal peptide fused to human V sequences, sequence variation in the signal peptide was found. Three patterns of changes in the signal peptide were observed. In the first pattern, the intact chicken signal peptide exon was spliced in-frame directly to the human V region exon, which was found in approximately 20% of the sequences in SynVH-SD and 5% in SynVH-C (Fig 2 and Table 2). The higher frequency of signal peptide changes in SynVH-SD suggests that there is a selection for such changes. Comparison of the signal peptide sequences in SynVH-C, SynVH-SD, and chicken VH showed that SynVH-SD contains a Lys residue in the central hydrophobic domain, where the SynVH-C contains Ile, which makes it less hydrophobic than either the SynVH-C signal peptide or the chicken VH signal peptide and a less efficient signal peptide (27) (Fig 2). The SynVH-SD signal peptide is identical to the human germline VH3-23 gene, whereas the SynVH-C signal peptide contains the Ile mutation relative to the germline. In the second pattern of signal peptide changes, the portion of the signal peptide encoded in the first exon was human but the 4 amino acids of the signal peptide that are encoded in the V exon were chicken (Fig 2). This pattern was observed in about 15% of the SynVH-SD sequences, but never in SynVH-C. This region is present in some of the chicken pseudogenes (8), so it is likely that gene conversion was responsible for mutating this region of the signal peptide to chicken. The chicken-derived sequence in this region extended slightly farther in these instances, to the third amino acid of the mature VH region (see below). In the third pattern of signal peptide changes, the SynVH-SD sequences retained the full human signal peptide, but carried a point mutation in the Lys residue (Fig 2), changing it to a hydrophobic residue (Val, Ile, or Met). This pattern occurred in about 40% of the SynVH-SD sequences. All three of these types of changes increased the hydrophobicity of the signal peptide. The high prevalence of these different types of changes to the SynVH-SD signal peptide strongly indicates positive selection for these changes.

**Table 2. SynVH-C but not SynVH-SD showed gene replacement by chicken VH, whereas both had changes in the signal peptide.**

| | Chicken VH | | Human VH | | huJH |
|---|---|---|---|---|---|
| | Ch sig pep | Hu sig pep | Ch sig pep | Hu sig pep | |
| SynVH-C | 333 (5.6%) | 5 (0.08%) | 318 (5.3%) | 5331 (88.9%) | 6000 (100%) |
| SynVH-SD | 0 | 0 | 613 (20.5%) | 2384 (79.5%) | 3000 (100%) |

The number (and percentage of total sequences from each transgene) of chicken and human VH regions is shown. Data from the six SynVH-C samples and three SynVH-SD samples were combined (total, 6000 sequences from SynVH-C birds and 3000 sequences from SynVH-SD birds). In the first set of columns are shown the sequences containing the chicken VH, split into those with a chicken signal peptide (Ch sig pep) or human signal peptide (Hu sig pep). In the second set of columns are the sequences with the human VH, similarly split for sequence of signal peptide. All of the sequences had the human JH (right column). The total number of VH regions is slightly less than the total number of sequences because a few sequences had VH deletions.

### Gene conversion in SynVH-C and SynVH-SD by chicken pseudogenes is rare

The chicken pseudogene array is still present upstream in both SynVH-C and SynVH-SD transgenes (Fig 1). In SynVH-SD, the chicken pseudogenes are in closer proximity to the human functional V, immediately upstream of the human pseudogenes, whereas in SynVH-C, the chicken V and D genes are between the two pseudogene arrays. As discussed above, the signal peptide sequence adjacent to FR1 underwent gene conversion in SynVH-SD, so it was interesting to know if further gene conversion of the human frameworks by chicken pseudogenes occurred. At the DNA level, the chicken germline VH segment is about 65% identical overall to the V genes in the transgenes. The longest stretches of homology are 11 bp, and mismatches of 1-6 bp are spread throughout the V regions. It is unclear whether this level and pattern of homology would be sufficient to enable gene conversion. To determine the functionally relevant levels of gene conversion that lead to changes in the protein sequence, FR1 and 3 were analyzed at the protein level for evidence of long stretches of chicken residue replacement (FR2 is too conserved between chicken and human to be able to unequivocally detect gene conversion events). Very low levels of gene conversion of the human FR1 and 3 in SynVH-C (0.07% in FR1 and 0.3% in FR3, in 5652 sequences) and SynVH-SD (2% in FR1 but none in FR3) were observed (Table 3). The most common example of gene conversion was the signal peptide change in SynVH-SD, which occurred in about 15% of the sequences, suggesting that if the sequence changes were under selection, then gene conversion could be observed at a higher frequency. The low frequencies of gene conversion events by chicken pseudogenes in the human FRs suggest that they are rare, non-selected events. The slightly higher frequency (2%) in SynVH-SD FR1 could be a result of a continuation of gene conversion events that began in the signal peptide region at the 5' end of the gene and continued into FR1. Another potential contributing factor could be the closer physical proximity on the chromosome of the human functional V to the chicken pseudogenes in SynVH-SD since the chicken V and D cluster were deleted in that transgene (Fig 1). In the antigen-specific mAb sequences derived from these birds (see below), no chicken-derived FR residues were seen in any sequence, which bolsters the idea that these rare gene conversion-derived sequences were not under selection nor were they necessary to produce antigen binders.

**Table 3. Gene conversion of human FRs by chicken pseudogenes is rare.**

| Sample | Signal Peptide* | FR1 | FR2 | FR3 | FR4 | # human seq |
|---|---|---|---|---|---|---|
| SynVH-C | | | | | | |
| 23806 | 0 | 2 | ND | 0 | 0 | 985 |
| 23824 | 0 | 1 | ND | 1 | 0 | 946 |
| 24317 | 0 | 1 | ND | 0 | 0 | 976 |
| 26934 | 0 | 0 | ND | 1 | 0 | 946 |
| 27022 | 0 | 0 | ND | 10 | 0 | 946 |
| 27023 | 0 | 0 | ND | 4 | 0 | 853 |

| SynVH-SD | | | | | | |
|---|---|---|---|---|---|---|
| 29406 | 172 | 8 | ND | 0 | 0 | 1000 |
| 29407 | 113 | 0 | ND | 0 | 0 | 1000 |
| 29409 | 174 | 60 | ND | 0 | 0 | 1000 |

The number of gene conversion events observed in the human sequences from each bird is given. Gene conversion is defined as a long tract of sequence (i.e. the whole of FR1) that is chicken sequence. The protein sequence was analyzed, not the nucleotide sequence. The number of human sequences analyzed is given at right.

ND: not determined. Chicken FR2 differs by only one (SynVH-SD) or two (SynVH-C) amino acids from the human transgene and this it was not possible to unequivocally assess gene conversion.
*the portion of the signal peptide encoded in the V region exon plus the first three amino acids of FR1, not the portion encoded in the first exon.

### CDR3 length diversity

CDR3 length diversity was analyzed in the two genotypes, from IMGT positions 105-117. In the SynVH-C sequences, the range was 3-22 amino acids, and the mean was 11.56 ± 2.06 (Fig 3). The CDR-H3 length in the germline of SynVH-C is a fixed length of 11 codons, since it is pre-rearranged, and any variation in length could only be a result of gene conversion or somatic hypermutation that deleted or inserted sequences. These mechanisms were more likely to increase the CDR length, as 55% have lengths longer than 11 residues, yet only 27% of the sequences have a length less than 11. In the rearranging transgene SynVH-SD, the mean CDR-H3 length was slightly longer and the distribution broader (11.89 ± 2.68 amino acids, range 3-21; neither set of length data fits a normal distribution) (Fig 3). Longer CDR-H3s were more frequent in SynVH-SD, with 23% of the CDRs of length 15 residues and above, as compared to 3.6% for SynVH-C. The hypothetical range of lengths in SynVH-SD, with no chewing back of coding sequences, would be 16-23 codons if a single D is used, indicating that most sequences undergo reduction in length either from chewing back during V(D)J recombination or from gene conversion/somatic hypermutation. Although the mAb sequences are limited and only for one antigen, the CDR-H3 lengths were skewed toward the longer lengths for SynVH-SD whereas SynVH-C frequencies seem to match the bulk sequencing (Fig. 3).

Chicken B cells lack TdT activity (9), so no additional nucleotides can be added during V(D)J rearrangement. In the WT human and chicken repertoires, CDR-H3s tend to be longer than those presented here, with mean CDR-H3 lengths of 15-16 residues, and a normal distribution (17,28,29). In WT chickens, the broad range of CDR-H3 lengths is produced by a combination of single D usage (the hypothetical range of lengths with no chewing back would be 20-22 codons if a single D is used), tandem D-D joining, exonucleolytic trimming, and gene conversion (6,8,29). In the rearranging SynVH-SD transgene these mechansims did not seem to produce the same lengths in the human V regions as in WT chicken antibodies. It is possible that the chicken Ds have been selected in evolution to prefer tandem D-D joins, which are common in chickens and are the mechanism to incorporate paired non-canonical cysteines to form intra-CDR3 disulfide bridges (see below). In contrast, D-D joins are not normally found in the human repertoire (29-31). Although no analysis was performed for D usage, the more limited CDR-H3 length found in the human sequences would suggest that D-D joins are not occurring, or if they are, there is selection against longer CDR-H3s produced by D-D joins of human genes in the chicken.

### Amino acid content in SynVH-C and SynVH- SD

Amino acid content of the CDRs was analyzed in the SynVH-C and SynVH-SD data sets (Table 4). The chicken V region sequences were removed and only the human sequences were analyzed. These data sets were based on immunized birds so they do not represent the naive repertoire, but the characteristics of the repertoires produced by the two transgenes may be compared to each other since the immunogen, human progranulin (PGRN), was the same for all birds. For CDR-H1 and H2 (IMGT definitions were used), all of the sequences were included regardless of length.

In CDR-H1, most of the amino acids were represented at similar frequencies in sequences from the two transgenes. One exception was Trp, which was not present in SynVH-C but found at 3.3% in SynVH-SD, all at IMGT position 38. The likely explanation is that Trp is found in three of the SynVH-SD pseudogenes, at position 38, but not in any of the SynVH-C pseudogenes. Similarly, Ala was more prevalent in SynVH-C sequences and reflects the presence of Ala in 15 of the 20 SynVH-C pseudogenes at IMGT position 38. Asp was more frequent in SynVH-SD, again most likely a result of gene conversion from pseudogenes containing Asp (7 out of 16 have at least one Asp in CDR1). Although clonal selection could alter frequencies of relevant amino acids, by increasing the frequency of residues involved in antigen binding to PGRN or having an effect on attributes such as expression level, at least some of the differences in frequencies should be attributable to differences in the rate of production of the changes in the first place.

In CDR-H2, amino acid distribution was also quite similar in the two genotypes, again with the biases usually traceable to residues found in the pseudogene pool. Trp was more frequent in SynVH-SD, and two of the SynVH-SD pseudogenes contain Trp, at IMGT position 58, whereas none of the SynVH-C pseudogenes contains Trp. For Trp to be present in SynVH-C sequences, it could only be caused by somatic hypermutation.

To compare CDR-H3 from SynVH-C and SynVH-SD transgenes, it was necessary to focus the analysis on the regions not contributed by the JH segment, since each transgene has a different JH germline gene. The JH6 gene used in SynVH-SD contains a string of 5 Tyr residues which biases the frequency of amino acids in the data toward Tyr and as a consequence reduces the frequency of the other amino acids if the whole CDR is included (regardless of whether the bounds are IMGT positions 105-117 or 107-114). The J gene in SynVH-C is JH4, which is shorter and contains only two Tyr residues. Therefore the focus was on CDR-H3 of specific lengths and the amino acid content for the positions normally contributed by the D region (positions 107-109 for 12-14 residue lengths, and 107-111 for 15 residues) were calculated. The average frequencies of each amino acid at these positions in CDR-H3s of those lengths are shown in Fig 4 and Table 4. In the portion of CDR-H3 that were analyzed, amino acid content was significantly different between SynVH-C and SynVH-SD sequences (χ² value 2047, 19 degrees of freedom, p<0.0001), and appeared more evenly distributed among the 20 amino acids in SynVH-SD sequences as compared to SynVH-C (Fig 4). (standard deviation was 4 times higher for SynVH-C than for SynVH-SD). Although many residues were found at similar frequencies in the two transgenes, there were a few notable exceptions. The most striking differences were serine (17.6% in SynVH-C compared to 2.9% in SynVH-SD) and glutamine (0.5% in SynVH-C compared to 6.3% in SynVH-SD). Tyrosine, an important component of the antigen contact site (32,33), was somewhat higher in SynVH-SD (3.8%) than in SynVH-C (2.0%). The Cys codons found in the human D2 family members were mutated in SynVH-SD to encode mainly Tyr, which could have increased the frequency of Tyr in the repertoire. Cysteine content is also higher in SynVH-SD (see below for details). Histidine frequency was much higher in SynVH-SD (5.8%) compared to SynVH-C (0.26%), although the overall frequencies of positively charged amino acids (K, R, H) were similar (SynVH-SD 13.0%, SynVH-C 11.7%). In CDR-H3, it was not possible to trace any of these differences in amino acid frequency to differences in the residues available in the pseudogene pool (Fig. 8). Ser, Gln and His residues can be found in the CDR-H3 regions of both pseudogene arrays.

To measure the amino acid variability in CDR-H3 of the two transgenes, Shannon entropy was calculated for each position in the subset of CDR3s of length 12-15 codons. Lengths of 12 and 15 residues are shown in Fig 5; 13 and 14 residues gave similar results. In the portion of the CDR-H3 loop mainly contributed by the D segment (positions 107-109 for CDRs of 12 residues and 107-111 for CDRs of 15 residues), diversity was similar for the two transgenes. Thus gene rearrangement and gene conversion alone can lead to similar levels of diversity in the regions not encoded by JH. In the regions that are encoded by JH, there was less diversity in SynVH-SD CDR-H3s, particularly the positions that are 5-7 residues from Trp-118 (such as positions 110, 112 and 113 in CDR-H3s of length 12 in Fig 5A). These positions are within the tandem stretch of tyrosines encoded by JH6, and tyrosine content was high at these positions in the sequencing data. However, other positions in the JH6 gene that encode tyrosine were found to be somatically mutated in SynVH-SD birds and could be highly diverse, such as positions 113 and 114. The lack of diversity at positions 112 and 112A may reflect a reduced involvement of these positions in antigen binding, resulting in less selection pressure for somatic mutation. The lack of specific CDR3 sequences in the SynVH-SD pseudogenes may also have been a contributing factor, although the variability in positions 108-111 and 113-114 was not reduced compared to SynVH-C despite those positions also lacking pseudogene donors.

To determine levels of diversity in the FR regions, Shannon entropy was calculated across the entire length of the V region (Fig. 9). FRs contained little diversity, in particular FR2. FR variability was somewhat lower in SynVH-SD than in SynVH-C, which may be expected since the pseudogenes in SynVH-C contain some FR changes whereas those in SynVH-SD do not. Any changes in the FRs in SynVH-SD must be from non-templated hypermutation, or potentially from chicken pseudogenes. The FR1 variability observed in SynVH-SD was partly from chicken pseudogenes (as discussed above), but the overall level of variability was still lower than that of SynVH-C in FR1. FR2 had essentially no variability.

The SynVH-C functional V region contains the motif RLF in FR3 (IMGT positions 90-92) that represents a somatic mutation compared to the germline residues QMN found in the VH3-23 gene. Several of the SynVH-C pseudogenes contain the QMN residues in those positions, enabling a reversion to the germline FR3 sequence by gene conversion. This reversion was observed in 94% of the sequences, strongly suggesting that the RLF motif was structurally disfavored and that the QMN residues were selected in the repertoire.

**Table 4. Amino acid distributions in CDRs 1-3.**

| | CDR1 (IMGT 27-38) | | CDR2 (IMGT 56-65) | | CDR3 (no FR or J), 12 - 15AA | |
|---|---|---|---|---|---|---|
| | SynVH-C | SynVH-SD | SynVH-C | SynVH-SD | SynVH-C | SynVH-SD |
| D | 5.24% | 12.16% | 5.04% | 4.44% | 14.05% | 10.76% |
| E | 0.05% | 0.00% | 0.59% | 0.28% | 6.24% | 3.47% |
| K | 0.12% | 0.15% | 0.26% | 1.16% | 3.88% | 1.40% |
| R | 1.53% | 0.57% | 2.43% | 1.54% | 7.58% | 5.76% |
| H | 2.68% | 0.71% | 0.60% | 0.32% | 0.26% | 5.80% |
| T | 12.11% | 11.98% | 12.80% | 14.07% | 8.18% | 8.05% |
| S | 17.70% | 14.85% | 23.92% | 23.46% | 17.61% | 2.87% |
| N | 3.80% | 3.10% | 5.02% | 9.72% | 4.86% | 9.21% |
| Q | 0.00% | 0.04% | 0.06% | 0.05% | 0.49% | 6.27% |
| G | 14.74% | 13.67% | 23.94% | 19.35% | 13.71% | 13.25% |
| A | 6.39% | 2.10% | 6.06% | 1.56% | 2.98% | 2.87% |
| C | 0.00% | 0.03% | 0.04% | 0.00% | 0.01% | 2.05% |
| P | 0.43% | 0.47% | 0.51% | 0.15% | 2.37% | 4.15% |
| V | 0.56% | 0.65% | 2.13% | 1.03% | 4.20% | 1.32% |
| I | 1.20% | 0.32% | 14.52% | 14.85% | 1.92% | 3.62% |
| L | 0.78% | 0.37% | 0.69% | 0.28% | 1.91% | 5.67% |
| M | 0.04% | 0.00% | 0.06% | 0.08% | 1.28% | 3.82% |
| F | 24.26% | 24.11% | 0.17% | 0.09% | 1.01% | 1.22% |
| Y | 8.35% | 11.43% | 1.14% | 3.39% | 1.96% | 3.82% |
| W | 0 | 3.27% | 0.04% | 4.17% | 5.49% | 4.62% |

IMGT CDR designations were used. For CDR-H3, amino acid frequencies in CDR lengths of 12 - 15 residues were calculated and averaged, removing residues encoded by V and J genes. Only the human sequences were analyzed from the 6 SynVH-C birds (n=5652) and 3 SynVH-SD birds (n=3000).

### Hydrophobicity of CDR3

Average hydrophobicity for each CDR-H3 (IMGT positions 105-117) from SynVH-C and SynVH-SD was calculated, based on the normalized Kyte-Doolittle scale of amino acid hydrophobicities (34,35). Data from CDR-H3 lengths of 12 - 15 residues were combined (Fig 6). The mean of these values falls on the hydrophilic side of the scale for both SynVH-C and SynVH-SD, and the range appears similar to that previously reported for human and mouse repertoires (36-38). The mean value for SynVH-SD CDR-H3s is shifted slightly toward the hydrophobic compared to SynVH-C (the difference in the mean of the hydrophobicity values was significant for the two groups (upaired Kolmogorov-Smirnov test, p<0.0001)).

### Cysteine content in FRs and CDRs

Low frequencies of non-canonical cysteine residues were found scattered throughout the V regions of SynVH-C and SynVH-SD sequences, either as single unpaired cysteines or pairs of cysteines that could potentially form disulfide bridges (Table 5). A total of 123 unpaired, individual cysteines were found in the 8652 VH sequences from the two transgenes (Table 5). These residues were found in all FRs and CDRs of both SynVH-C and SynVH-SD, including CDR-H3, except for FR1 of SynVH-C. The frequency of unpaired cysteines was lower than that observed in WT chickens (0.08, 0.13 and 0.17% vs. 2.1, 0.8 and 2.4% in CDR1, FR2 and CDR2 from SynVH vs. WT (17)).

In CDR-H3, the total cysteine content in SynVH-C sequences was 0.01%, and in SynVH-SD, 2.05% (Table 4), compared to the 1.21% reported in humans (28). The occurrence of cysteine in CDR-H3 is thus similar between human sequences derived from chickens or humans. In addition to the unpaired cysteines, a small number of paired cysteines that could form disulfide bridges was also observed within CDR-H3 of both transgenes (3 sequences in SynVH-C, and 110 sequences in SynVH-SD) (Tables 4 and 5). SynVH-C also had 6 instances of paired cysteines in CDR-H2, but SynVH-SD had none. These paired cysteines form potential disulfide-stabilized loops, but there is very little diversity in the sequences of the loops themselves. Only two unique sequences each from SynVH-C and SynVH-SD were found, indicating that these paired cysteines only occurred rarely and then spread by clonal expansion within their family trees. It was striking that SynVH-SD contained more instances of potential disulfide loops in CDR-H3 than SynVH-C (3.7% of the SynVH-SD sequences contained a potential intra-CDR3 disulfide loop compared to 0.05% of SynVH-C sequences). Although the SynVH-SD transgene had the D cluster which could in principle provide higher cysteine content, the transgene was designed such that the Cys codons normally found in the germline human D2 family members were all mutated to encode Tyr or Trp. In the sequences of the SynVH-SD pseudogenes, the region downstream of the FR3 does contain some Cys codons, so gene conversion (as well as somatic hypermutation) could have added cysteines. The SynVH-SD pseudogene design was based on the germline VH3 family CDRs, and since germline V genes do not contain CDR3, the region downstream of FR3 in the pseudogenes was simply a spacer sequence between pseudogenes. These spacers could provide a potential source of diversity if they are used in gene conversion.

The cysteine content of the human sequences from chickens is low, as in humanderived sequences, which is in sharp contrast to the cysteine content of normal chicken antibodies (17). In WT chickens, 53% of non-selected VH clones contained two non-canonical cysteines in CDR-H3, compared to 1.3% of the human sequences from chickens. These paired cysteines may form small loops that stabilize the antigen-binding structure in chicken CDR-H3s. The cysteines are separated by 2-4 amino acids in the human sequences presented here, whereas in some cases the loops in chicken antibodies can be longer (17). Chicken VH sequences often contain a single cysteine in CDR3 and a second cysteine elsewhere in the VH region, forming a potential disulfide bridge from CDR3 to another part of the V region (Types 3-6 of Wu et al.). No such paired cysteines were observed in the human sequences from the chicken. Only one sequence out of 8652 from both transgenes had two non-canonical cysteines in different parts of the V region, a single SynVH-C sequence with cysteines in FR2 and FR3. This particular pattern was not seen in chickens (17).

**Table 5. Non-canonical cysteine content is low in SynVH-C and SynVH-SD birds.**

| | | FR1 | CDR1 | FR2 | CDR2 | FR3 | CDR3 | FR4 |
|---|---|---|---|---|---|---|---|---|
| SynVH-C | Single Cys | 0 | 2 | 8 | 13 | 5 | 43 | 9 |
| | Paired Cys | 0 | 0 | 0 | 4 (CDSC) | 0 | 3 (CWNFLC) | 0 |
| | | | | | 2(CC) | | | |
| SynVH-SD | Single Cys | 3 | 5 | 3 | 2 | 2 | 22 | 6 |
| | | | | | | | | |
| | Paired Cys | 0 | 0 | 0 | 0 | 0 | 34 (CNDYYC) | 0 |
| | | | | | | | 76 (CYYC) | |

Numbers refer to the number of unique sequences containing a single, unpaired Cys residue or two potentially paired Cys residues in the top 1000 unique sequences from each bird (only human V regions were analyzed; total, n=5652 for SynVH-C and 3000 for SynVH-SD). In parentheses are shown the sequences of the potential loops. The only other potential disulfide loop was a single SynVH-C sequence with Cys residues in FR2 and FR3. In FR2 and FR4, all instances of non-canonical Cys were cases of Trp->Cys changes, which can occur by a single nucleotide substitution. No instances of more than 2 non-canonical cysteines were observed.

### Comparison of mAbs to NGS data

The spleen lymphocytes that were the source of the NGS data came from birds that had been immunized and used to produce antigen-specific antibodies. The six SynVH-C and three SynVH-SD birds were immunized with a test immunogen, human progranulin (PGRN). Panels of antigen-specific mAbs were identified from each bird, by screening spleen cells in GEM assays (18,39). The mAbs were confirmed as binding to PGRN by ELISA, and sequences of the heavy and light chain V regions were obtained. The light chain in all of these birds was provided by a human V-kappa transgene (18). The birds were heterozygous for both heavy and light chain transgenes, with light and heavy chain knockouts on the other alleles. Therefore only human V-region antibodies are produced in the birds.

The unique VH sequences of the antigen-specific mAbs from each individual bird were compared to the NGS data from the same bird (total, 177 mAbs). The number of times each mAb was sequenced in the NGS data is shown in Fig 7. All 177 mAb sequences were found in the NGS data, with varying degrees of matching. 79 of the mAbs (45%) were sequenced 5 times or fewer in the NGS data, and 37 were only sequenced once, indicating that GEM screens can readily identify rare mAbs. If one were to select mAbs based on sequence data alone, one would likely miss these rare mAbs. Others were found multiple times, up to about 7000 times. About one-third (63/177) of the mAb sequences matched perfectly to a unique peptide sequence in the NGS data, whereas the rest had some changes relative to the closest match. These changes probably reflect the fact that the NGS data represent a snapshot of the spleen, at most about 10⁶ B cells (on average, the total number of sequences from each bird that were obtained), whereas the mAbs were derived from a GEM screen of a larger sample size, about 7x106 B cells. The sequence from a single cell picked in a GEM screen might be related to sequences in a family tree in the NGS data although the exact sequence was not found in the NGS data. Sequence errors introduced by reverse transcription or library preparation could also be a factor.

### Discussion

The chicken gene conversion/somatic hypermutation system has evolved to produce a diverse repertoire that is capable of conferring protective immunity on its host. The lack of diversity from combinatorial mechanisms does not seem to be a limitation for the CDR-H3 repertoire. When two transgenes encoding human variable regions were introduced, one which cannot undergo rearrangement but is forced to generate its sequence diversity purely through gene conversion and/or somatic hypermutation, and the other which undergoes rearrangement and has the choice of all human Ds to produce combinatorial diversity, the levels of amino acid diversity were similar. Because the dataset came from immunized birds, there could be some bias toward specific residues from the immunization, but the two transgenes behaved similarly despite having different modes of diversity generation. In CDRs 1 and 2, and the FRs, it was possible to correlate amino acid frequencies to residues found in the human pseudogenes in both transgenes, but for CDR3, it was not.

The range and mean of CDR-H3 lengths were also quite similar in both the pre-rearranged and rearranging transgenes. It is striking that the mean CDR-H3 lengths were so similar in the two transgenes given that the rearranging transgene in principle could produce a much wider range from variation in D usage. The lack of TdT in chickens limits the lengths to what is encoded by the D genes, or what could be inserted by gene conversion, which could explain the shorter lengths seen here compared to the normal human repertoire. Gene conversion alone may not be able to produce as wide a range of CDR-H3 lengths as rearrangement with TdT activity (as in humans), or rearrangement with D-D joins (as in WT chickens). SynVH-SD did not produce the range of lengths that were theoretically possible by D-D joins or even single D rearrangements. Constraints on CDR-H3 length could come from pairing with the particular V-kappa light chain in the transgenic chickens, potentially unfavorable structural attributes of such human D-D sequences, or unknown cis-acting sequences that promote D-D joins in chicken that were not included in the human SynVH-SD construct. Even though the range and mean CDR-H3 lengths were similar, SynVH-SD did contain a significant proportion of longer CDR-H3s (15 residues and above) that could represent an advantage in an antibody discovery program, as there may be more opportunity for added functionality, such as broader epitope coverage, agonistic or antagonistic functions, and kinetics. It has recently been shown that cow antibodies with ultra-long (~60 amino acid) H3 domains can be potent neutralizers across many clades of HIV, possibly due to their ability to bind conserved, but occluded, epitopes on the virus (40).

Structures that are commonly found in the chicken, such as non-canonical disulfide bridges, were extremely rare in the human sequences. The human germline sequences and pseudogenes that are available during repertoire development do not encode such structures, so there is no obvious mechanism to produce them. To obtain high affinity human antibodies in the chicken, these structures are not necessary since the mAbs compared in this study had a range of affinities down to 0.11 nM, and epitope coverage similar to that in WT chickens (18). These data are supported by the observation that mAbs from WT chickens recognizing novel epitopes do not require a disulfide bridge although they are frequently present (41).

Selection for functional sequences was clearly in effect for the germline VH3-23 signal peptide in SynVH-SD and for the QMN motif in FR3 of SynVH-C. The germline VH3-23 gene contains a lysine residue in the signal peptide, which was mutated (to Ile) in the human somatically-derived SynVH-C signal peptide. Multiple mechanisms came into play to mutate the SynVH-SD signal peptide sequence in chickens, including gene conversion at the 5' end of the V gene. This result shows that gene conversion of chicken pseudogenes can act on the human functional V, when it could provide a useful purpose. Despite the ability for gene conversion from chicken pseudogenes to act on the human V region, very little gene conversion of the human FRs was observed in the bulk sequencing, and in the mAb sequences, no chicken gene conversion was found at all. From the standpoint of the gene conversion mechanism, the chicken pseudogenes could be at a disadvantage because of their physical distance from the human functional V and their reduced level of homology (5,11,13). This could explain the lack of gene conversion by chicken pseudogenes. From a functional standpoint, the diversity that is achievable by the human pseudogenes and somatic hypermutation is extensive enough to enable antibodies capable of binding antigen, so in general there is no selective pressure driving further mutation by the chicken pseudogenes.

### EXAMPLE 2

### Creation of a VDJ knock out

Guide RNA design: A 159bp region upstream of the chicken functional heavy chain V was analyzed on the MIT server for guide RNA design. Four guide RNAs were selected, synthesized and cloned separately into the GE6 vector containing the wild type Cas9 nuclease (Horizon): gRNA1, AAATCATTAATCAACCCGAC (SEQ ID NO: 43); gRNA2, AACACGACTCCGGGCCTAGA (SEQ ID NO: 44); gRNA3, TGATTAATTGGGCGCCCGTC (SEQ ID NO: 45); gRNA4, ATTTAATGGCCGTCTAGGCC (SEQ ID NO: 46). These gRNAs had few predicted offtarget sites, none of which were in known coding sequences. A control construct containing gRNA5 specific for EGFP was also made (AAGTTCGAGGGCGACACCC (SEQ ID NO: 47)).

Targeting vector IgH KO6B design: Homology regions of 1133bp and 1011bp were PCR amplified and cloned from a homozygous knockout chicken that carried the original JH-KO (Schusser et al Proc Natl Acad Sci USA. 2013 110: 20170-20175). The 5' HR was amplified with primers 5'-GCCCCTAATAAGTGGTTTAATTATG-3' (SEQ ID NO: 48) and 5'-TCTGCGCTGAGTTCTTTGAT-3' (SEQ ID NO: 49); the 3' HR was amplified with primers 5'-AAGTCGAGGCTGACGAGAAA-3' (SEQ ID NO: 50) and 5'-CTTTTCCCCACCAAATTTCA-3' (SEQ ID NO: 51). Homozygous DNA ensured that the homology regions would be isogenic to the allele carrying the JH-KO in the cells used for targeting, 472-138, which are heterozygous for the JH-KO. The two alleles are likely to be polymorphic since the chickens used to derive these PGCs are outbred. In the IgH locus, the homology regions are separated by a stretch of 122 bp, which contains the sequences targeted by the gRNAs, ensuring that the gRNAs will target only the genome and not the targeting vector itself when they are cotransfected into the cells. The homology regions flank a β-globin HS4-insulated hygromycinresistance gene for selection in PGCs and a loxP site designed to recombine with the downstream loxP sites in the JH-KO selectable marker cassette.

Cells used for targeting: PGC line 472-138 contains a previously targeted heavy chain locus in which the JH region (Schusser et al Proc Natl Acad Sci USA. 2013 110: 20170-20175) was replaced with a floxed selectable marker cassette. The cell line was derived by breeding a germline chimeric male, which had been injected with JH-KO PGCs, to a wild type hen and culturing cells from the germinal crescent of a Stage 4-8 (Hamburger and Hamilton) EGFP-positive embryo. PGCs were cultured as described [6]. Briefly, PGCs were grown in KO-DMEM (Life Technologies), of which 40% was preconditioned on buffalo rat liver cells (BRL, ATCC), and supplemented with 7.5% fetal bovine serum (Hyclone), 2.5% irradiated chicken serum, 1X non-essential amino acids, 2mM glutamine, 1mM sodium pyruvate, 0.1mMβ-mercaptoethanol (all from Life Technologies), 4ng/ml recombinant human fibroblast growth factor, 6ng/ml recombinant mouse stem cell factor (both from R&D Systems) and grown on an irradiated feeder layer of BRL cells. The cells were passaged 3 times per week onto fresh feeder layers.

Transfection and injection: To test for inactivation of the EGFP locus, 15µg of either EGFP-specific gRNA5/Cas9 or Cas9 alone were added to 3 x 106 cells and brought to a volume of 100µl with V-buffer (Lonza, Walkersville). The cell suspension was transferred to a 2mm cuvette and subjected to 8 square wave pulses of 350 volts/100µsec (BTX 830 electroporator). After electroporation the cells were resuspended in culture medium and cultured for 9 days to allow the remaining EGFP in the cells to dilute out. Cells were analyzed for loss of green fluorescence using the Attune flow cytometer (Life Technologies). For stable transfectants targeting the IgH locus, 15µg of circular gRNA1, 2, 3 or 4/Cas9 and 2.5, 5 or 15µg circular IgH KO6B were added to 5x106 cells and transfected as described above, then plated with hygromycin-resistant irradiated BRLs and seeded in a 48-well plate at a density of 105 cells per well. A control transfection with 5µg IgH KO6B without gRNA/Cas9 was also done. After 3 days, 40µg/ml hygromycin was added to select for cells with a stable integration of IgH KO6B. After stable clones were identified, the cells were expanded and confirmed for the IgH KO6B integration by PCR. Confirmed clones were injected into recipient chicken embryos at Stage 14-16 (H&H). The injected embryos were transferred to surrogate shells and incubated until hatch at 37°C. The sex of the chicks was determined after hatch by PCR for the W-chromosome.

Screening for targeting by IgH KO6B: Hygromycin-resistant clones were analyzed by PCR for targeting by IgH KO6B. For the 5' assay, the forward primer was chVH-F5: 5'-TGGTTTGGTTGATGGAAGAATGTA-3' (SEQ ID NO: 52) and the reverse primer was HA-R: 5'-ATACGATGTTCCAGATTACGCTT-3' (SEQ ID NO: 53). For the 3' assay, the forward primer was KO 6B-F: 5'-GCTGAACTAGAATGCATCAAGC-3' (SEQ ID NO: 54) and reverse primer chVH-R33: 5'-ACAAACCTTTGCCGCATCCA-3' (SEQ ID NO: 55).

Cre recombination of inserted loxP site in the IgH locus: 3x10⁶ Cells from line 1783-9, carrying the CRISPR-targeted loxP site and JH-KO loxP sites, were transiently transfected with 20µg of a β-actin-Cre expression construct as described above, and cultured for 10 days. The Cre transfection was then repeated to increase the percentage of cells with the excision, and four days later the cells were harvested for PCR analysis of Cre/lox recombination between the two outermost loxP sites. Two PCR assays were performed: both used a 5' primer in the upstream VH flanking region (chVH-F3aB: 5'-GATG GGGGGTGGCAATGGAATGAT-3' (SEQ ID NO: 56)). The 3' primer was located either in the neo gene in the JH-KO (neo-F1: 5'-AGCTGTGCTCGACGTTGTCACT-3' (SEQ ID NO: 57)) generating a 1.6kb amplicon, or in the IgH locus downstream of the selectable markers (chJC-R45: 5'-GCCCAAAATGGCCCCAAAAC-3' (SEQ ID NO: 58)), generating a 2kb amplicon.

To test whether CRISPR/Cas9 could be used to edit the PGC genome, experiements were first performed to inactivate an enhanced green fluorescent protein (EGFP) transgene inserted in the IgH locus. The EGFP gene is part of a selectable marker cassette that was used to knock out the JH gene segment in the JH-KO PGC cell line 472-138 (Fig 10, A). A previously described gRNA (gRNA5) to EGFP was cloned in a U6-expression vector (GE6) that also carries the wild type Cas9 nuclease. PGCs were transiently transfected with constructs encoding Cas9 with and without gRNA5. Nine days after transfection, the cell population was analyzed by flow cytometry, and ~9% of the cells in the Cas9/gRNA5 population had lost EGFP expression compared to the control transfection (Fig 10, B).

To produce genetically-modified birds with CRISPR/Cas9, the modification produced in PGCs must be able to be passed through the germline to the next generation. For this purpose, clonal populations in which every cell carries the desired mutation are preferred. A drug selection strategy was designed in combination with CRISPR/Cas9 to select and grow clones carrying the modification. The region upstream of the single immunoglobulin heavy chain variable region (VH) in the JH-KO cells was targeted for the introduction of a loxP site into the IgH locus. Four guide RNAs were designed to direct double-strand cutting of the genome by Cas9 at a site approximately 300bp upstream of the translation initiation site of the VH (Fig 11, A) and each one was cloned separately into the GE6 vector with Cas9. The Cas9 cut sites are all approximately 50bp from the homology regions in the donor targeting vector, IgH KO6B. IgH KO6B was constructed with short homology regions of ~1kb flanking a hygromycin selection cassette (Fig 11, A).

Although the chicken IgH locus consists of only short stretches of sequence in the genome database, not organized into contigs, there was sufficient unique sequence available to design PCR primers and amplify these short homology regions. The homology regions were amplified from homozygous JH-KO genomic DNA and are thus isogenic for the allele that contains the JH-KO in the 472-138 cell line. The other allele in these cells is likely to be polymorphic since the bird used for deriving cell line 472-138 was outbred.

The 4 gRNAs to VH were separately co-transfected with circular IgH KO6B and stable transformants were selected with hygromycin. In the first set of transfections, we used 15µg of each plasmid (IgH KO6B and gRNA/Cas9) for 5 x 106 cells, amounts of DNA that would normally yield approximately 1-10 colonies per 48-well plate when using linearized targeting vectors alone. However, when IgH KO6B was used in combination with gRNA/Cas9, the transfections were so efficient that every well for all 4 transfections contained multiple clones of hygromycin-resistant cells. Although these were not clonal populations, 3 wells from each transfection were harvested to test for targeting by IgH KO6B, and all contained the correct targeting event (Fig. 11, B). With gRNAs 1, 3 and 4, most wells had perhaps 4-5 colonies per well, suggesting that the targeting efficiency was, at worst, 20-25% (if there were only 1 positive clone out of 4-5). With gRNA2, most wells only had ~2-3 clones, suggesting a higher potential efficiency of ~33%, so for subsequent transfections we used gRNA2, and used lower amounts of the donor IgH KO6B to reduce the number of resistant colonies. Transfection with 2.5µg IgH KO6B + gRNA2/Cas9 yielded 12 colonies per 48-well plate, 9 of which were screened for targeting. All 9 clonal populations had the correct targeting (Fig. 11, C). Transfection with 5µg of IgH KO6B + gRNA2/Cas9 yielded >50 clones, while a control transfection with 5µg IgH KO6B without the gRNA/Cas9 yielded only 2 colonies, neither of which was targeted correctly (data not shown).

Targeting of the VH region was confirmed independently, taking advantage of the loxP site placed adjacent to the hygromycin gene in the targeted allele. The downstream JH-KO selectable markers are flanked by loxP sites (floxed) and should be on the same chromosome as the VH loxP site. If the targeting is correct the Cre recombination should excise the intervening DNA and leave behind a single loxP site and the promoterless neo gene (Fig 12, A). Cells of the CRISPR-targeted clone 1783-9 were transiently transfected with a Cre-expression construct two times sequentially (to increase the percentage of cells with the excision) and cells were grown for four days after the second transfection to allow recombination. PCR primers lying outside of the loxP sites amplified the expected size products, whereas in unrecombined cells the primers are about 28kb apart, and no product was observed (Fig. 12, B), indicating that the CRISPR-targeted VH loxP site was in the correct position and orientation.

Five targeted cell lines (1783-1, 3, 6, 9 and 10) were injected into embryos to create germline chimeras. Male chimeras were bred to wild type females and germline progeny screened by green fluorescence, using the EGFP transgene in the JH-KO selectable marker cassette. Four of the 5 cell lines transmitted germline progeny, to varying degrees. One cell line, 1783-10, exhibited high rates of germline transmission, including one chimera with close to 100% transmission of the injected cells. Forty-six EGFP-positive progeny from transmission of cell line 1783-10 were hatched and typed for the CRISPR-targeted IgH KO6B. All of the EGFP positive birds contained the IgH KO6B insertion, confirming that the PGC clones contained the correct stable integration.

### REFERENCES

1. Ratcliffe MJH. Antibodies, immunoglobulin genes and the bursa of Fabricius in chicken B cell development. Dev Comp Immunol (2006) 30:101-118. doi:10.1016/j.dci.2005.06.018
2. Pike K, Ratcliffe M. Ligand-independent signaling during early avian B cell development. Immunol Res (2006) 35:103-116.
3. Aliahmad P, Pike KA, Ratcliffe MJH. Cell surface immunoglobulin regulated checkpoints in chicken B cell development. Vet Immunol Immunopathol (2005) 108:3-9. doi:10.1016/j.vetimm.2005.08.009
4. McCormack W, Tjoelker L, Carlson L, Petryniak B, (null), Humphries E, Thompson C. Chicken IgL gene rearrangement involves deletion of a circular episome and addition of single nonrandom nucleotides to both coding segments. Cell (1989) 56:785-91.
5. Reynaud C, Anquez V, Dahan A, Weill J. A single rearrangement event generates most of the chicken immunoglobulin light chain diversity. Cell (1985) 40:283.
6. Reynaud C, Anquez V, Weill J. The chicken D locus and its contribution to the immunoglobulin heavy chain repertoire. Eur J Immunol (1991) 21:2661-70.
7. Benatar T, Tkalec L, Ratcliffe MJ. Stochastic rearrangement of immunoglobulin variable-region genes in chicken B-cell development. Proc Natl Acad Sci USA (1992) 89:7615-7619.
8. Reynaud C, Dahan A, Anquez V, Weill J. Somatic hyperconversion diversifies the single VH segment of the chicken with a high incidence in the D region. Cell (1989) 59:171-83.
9. Yang B, Gathy KN, Coleman MS. T-cell specific avian TdT: characterization of the cDNA and recombinant enzyme. Nucleic Acids Res (1995) 23:2041-2048.
10. Mansikka A, Sandberg M, Lassila O, Toivanen P. Rearrangement of immunoglobulin light chain genes in the chicken occurs prior to colonization of the embryonic bursa of Fabricius. Proc Natl Acad Sci USA (1990) 87:9416-9420.
11. Reynaud C-A, Anquez V, Grimal H, Weill J-C. A hyperconversion mechanism generates the chicken light chain preimmune repertoire. Cell (1987) 48:379-388. doi:10.1016/0092-8674(87)90189-9
12. Carlson LM, McCormack WT, Postema CE, Humphries EH, Thompson CB. Templated insertions in the rearranged chicken IgL V gene segment arise by intrachromosomal gene conversion. (1990) 4:536-47.
13. McCormack WT, Thompson CB. Chicken IgL variable region gene conversions display pseudogene donor preference and 5" to 3" polarity. Genes Dev (1990) 4:548-558.
14. Parvari R, Ziv E, Lantner F, Heller D, Schechter I. Somatic diversification of chicken immunoglobulin light chains by point mutations. Proc Natl Acad Sci USA (1990) 87:3072-3076.
15. Arakawa H, Saribasak H, Buerstedde J. Activation-induced cytidine deaminase initiates immunoglobulin gene conversion and hypermutation by a common intermediate. PLoS Biol (2004) 2:E179.
16. Sale J, Calandrini D, Takata M, Takeda S, Neuberger M. Ablation of XRCC2/3 transforms immunoglobulin V gene conversion into somatic hypermutation. Nature (2001) 412:921-926.
17. Wu L, Oficjalska K, Lambert M, Fennell BJ, Darmanin-Sheehan A, Ní Shúilleabháin D, Autin B, Cummins E, Tchistiakova L, Bloom L, et al. Fundamental characteristics of the immunoglobulin VH repertoire of chickens in comparison with those of humans, mice, and camelids. J lmmunol (2012) 188:322-333. doi:10.4049/jimmunol.1102466
18. Ching KH, Collarini EJ, Abdiche YN, Bedinger D, Pedersen D, Izquierdo S, Harriman R, Zhu L, Etches RJ, van de Lavoir M-C, et al. Chickens with humanized immunoglobulin genes generate antibodies with high affinity and broad epitope coverage to conserved targets. MAbs (2017) doi:10.1080/19420862.2017.1386825
19. Leighton PA, Schusser B, Yi H, Glanville J, Harriman W. A Diverse Repertoire of Human Immunoglobulin Variable Genes in a Chicken B Cell Line is Generated by Both Gene Conversion and Somatic Hypermutation. Front Immun (2015) 6:126. doi: 10.3389/fimmu.2015.00126
20. Tiller T, Schuster I, Deppe D, Siegers K, Strohner R, Herrmann T, Berenguer M, Poujol D, Stehle J, Stark Y, et al. A fully synthetic human Fab antibody library based on fixed VH/VL framework pairings with favorable biophysical properties. MAbs (2013) 5:445-470. doi:10.4161/mabs.24218
21. Schusser B, Collarini EJ, Yi H, Izquierdo SM, Fesler J, Pedersen D, Klasing KC, Kaspers B, Harriman WD, van de Lavoir MC, et al. Immunoglobulin knockout chickens via efficient homologous recombination in primordial germ cells. Proc Natl Acad Sci USA (2013) 110:20170-20175. doi:10.1073/pnas.1317106110
22. Dimitrov L, Pedersen D, Ching KH, Yi H, Collarini EJ, Izquierdo S, van de Lavoir M-C, Leighton PA. Germline Gene Editing in Chickens by Efficient CRISPR-Mediated Homologous Recombination in Primordial Germ Cells. PLoS ONE (2016) 11:e0154303. doi:10.1371/journal.pone.0154303
23. Chen C, Nagy Z, Prak EL, Weigert M. Immunoglobulin heavy chain gene replacement: a mechanism of receptor editing. Immunity (1995) 3:747-755.
24. de Wildt R, Hoet R, van Venrooij W, Tomlinson I, Winter G. Analysis of heavy and light chain pairings indicates that receptor editing shapes the human antibody repertoire. J MolBiol (1999) 285:895-901.
25. Cascalho M, Ma A, Lee S, Masat L, Wabl M. A quasi-monoclonal mouse. Science (1996) 272:1649-1652.
26. Briney BS, Crowe JE. Secondary mechanisms of diversification in the human antibody repertoire. Front Immun (2013) 4:42. doi:10.3389/fimmu.2013.00042
27. Haryadi R, Ho S, Kok YJ, Pu HX, Zheng L, Pereira NA, Li B, Bi X, Goh L-T, Yang Y, et al. Optimization of heavy chain and light chain signal peptides for high level expression of therapeutic antibodies in CHO cells. PLoS ONE (2015) 10:e0116878. doi:10.1371/journal.pone.0116878
28. Zemlin M, Klinger M, Link J, Zemlin C, Bauer K, Engler JA, Schroeder HW, Kirkham PM. Expressed murine and human CDR-H3 intervals of equal length exhibit distinct repertoires that differ in their amino acid composition and predicted range of structures. J MolBiol (2003) 334:733-749.
29. Larimore K, McCormick MW, Robins HS, Greenberg PD. Shaping of human germline IgH repertoires revealed by deep sequencing. The Journal of Immunology (2012) 189:3221-3230. doi:10.4049/jimmunol.1201303
30. Ohm-Laursen L, Nielsen M, Larsen SR, Barington T. No evidence for the use of DIR, D-D fusions, chromosome 15 open reading frames or VH replacement in the peripheral repertoire was found on application of an improved algorithm, JointML, to 6329 human immunoglobulin H rearrangements. Immunology (2006) 119:265-277. doi:10.1111/j.1365-2567.2006.02431.x
31. Corbett SJ, Tomlinson IM, Sonnhammer EL, Buck D, Winter G. Sequence of the human immunoglobulin diversity (D) segment locus: a systematic analysis provides no evidence for the use of DIR segments, inverted D segments, "minor" D segments or D-D recombination. J MolBiol (1997) 270:587-597. doi:10.1006/jmbi.1997.1141
32. Collis AVJ, Brouwer AP, Martin ACR. Analysis of the antigen combining site: correlations between length and sequence composition of the hypervariable loops and the nature of the antigen. J Mol Biol (2003) 325:337-354.
33. Ofran Y, Schlessinger A, Rost B. Automated identification of complementarity determining regions (CDRs) reveals peculiar characteristics of CDRs and B cell epitopes. The Journal of Immunology (2008) 181:6230-6235.
34. Kyte J, Doolittle R. A simple method for displaying the hydropathic character of a protein. J Mol Biology (1982) 157:105-132.
35. Eisenberg D. Three-dimensional structure of membrane and surface proteins. Annu Rev Biochem (1984) 53:595-623. doi:10.1146/annurev.bi.53.070184.003115
36. Ivanov II, Schelonka RL, Zhuang Y, Gartland GL, Zemlin M, Schroeder HW. Development of the expressed Ig CDR-H3 repertoire is marked by focusing of constraints in length, amino acid use, and charge that are first established in early B cell progenitors. J Immunol (2005) 174:7773-7780.
37. Mroczek ES, Ippolito GC, Rogosch T, Hoi KH, Hwangpo TA, Brand MG, Zhuang Y, Liu CR, Schneider DA, Zemlin M, et al. Differences in the composition of the human antibody repertoire by B cell subsets in the blood. Front Immun (2014) 5:96. doi: 10.3389/fimmu.2014.00096
38. Rogosch T, Kerzel S, Hoi KH, Zhang Z, Maier RF, Ippolito GC, Zemlin M. Immunoglobulin analysis tool: a novel tool for the analysis of human and mouse heavy and light chain transcripts. Front Immun (2012) 3:176. doi:10.3389/fimmu.2012.00176
39. Mettler-Izquierdo S, Varela S, Park M, Collarini EJ, Lu D, Pramanick S, Rucker J, Lopalco L, Etches R, Harriman W. High-efficiency antibody discovery achieved with multiplexed microscopy. Microscopy (Oxf) (2016) 65:341-352. doi:10.1093/jmicro/dfw014
40. Sok D, Le KM, Vadnais M, Saye-Francisco K, Jardine JG, Torres J, Berndsen ZT, Kong L, Stanfield R, Ruiz J, et al. Rapid elicitation of broadly neutralizing antibodies to HIV by immunization in cows. Nature (2017) doi:10.1038/nature23301
41. Könitzer JD, Pramanick S, Pan Q, Augustin R, Bandholtz S, Harriman W, Izquierdo S. Generation of a highly diverse panel of antagonistic chicken monoclonal antibodies against the GIP receptor. MAbs (2017) 9:536-549. doi:10.1080/19420862.2016.1276683

## Claims

1. A transgenic chicken, wherein the transgenic chicken comprises a genome comprising a modified endogenous immunoglobulin heavy chain (IgH) locus that:
(a) lacks the entire contiguous endogenous chicken V-D-J region; and
(b) comprises, in operable linkage:
(i) an immunoglobulin heavy chain gene promoter;
(ii) a germline human V_{H} segment comprising a coding sequence for a variable domain that comprises FR1, CDR1, FR2, CDR2, and FR3 sequences;
(iii) a human D cluster;
(iv) a single human J segment;
(v) a plurality of sequences encoding constant regions; and, upstream of the germline human VH segment of (b)(ii):
(vi) a plurality of pseudogenes of structure FR1-CDR1-FR2-CDR2-FR3 that each comprise:
FR1, FR2 and FR3 sequences that are at least 95% identical to or identical to the FR1, FR2 and FR3 sequences in the functional human VH segment of (b)(ii); and
CDR1 and CDR2 sequences that differ from pseudogene to pseudogene,
wherein:
the modified IgH locus undergoes VDJ recombination in B-cells of the chicken;
the plurality of germline human V_{H} pseudogenes, after VDJ recombination, donate nucleotide sequences to the germline human VH segment of b(ii) by gene conversion; and
the chicken produces humanized antibodies that comprise a diversified immunoglobulin human heavy chain variable domain.

2. The transgenic chicken of claim 1, wherein the CDR1 and CDR2 sequences of (b)(vi) that encode the CDR1s and CDR2s of different human VH segments that are in the same family as the functional human VH segment of (b)(ii).

3. The transgenic chicken of any prior claim, wherein the entire contiguous endogenous chicken V-D-J region of (a) in the range of 10-12 kb in length.

4. The transgenic chicken of any prior claim, wherein the promoter of (b)(i) is a chicken immunoglobulin heavy chain gene promoter.

5. The transgenic chicken of any prior claim, wherein the germline human VH segment of (b)(ii) and the CDR1 and CDR2 sequences of (vi) are from the V_{H}3 family, the V_{H}1 family or the V_{H}4 family.

6. The transgenic chicken of any prior claim, wherein, in the D cluster, any codons for a cysteine have been mutated to encode another amino acid and, optionally, wherein, in the D cluster, any codons for a cysteine have been mutated to encode tyrosine or tryptophan.

7. The transgenic chicken of any prior claim, wherein the intervening sequences in the D cluster are from chicken.

8. The transgenic chicken of any prior claim, wherein the plurality of pseudogenes of (b)(v) comprises at least 10 of the pseudogenes, wherein the pseudogenes of (b)(v) are in reverse orientation to the germline human VH segment of (b)(ii), and/or wherein the plurality of sequences encoding constant regions of (b)(v) are endogenous to the chicken.

9. The transgenic chicken of any prior claim, wherein the transcript of the modified immunoglobulin heavy chain (IgH) locus comprises an intron that joins the 3' end of a copy of the J coding sequence to the 5' end of a copy of the constant region coding sequence of (b)(v).

10. The transgenic chicken of any prior claim, wherein the chicken is homozygous for the modified IgH locus, wherein the chicken is heterozygous for the modified IgH locus and the IgH locus on the homologous chromosome is knocked out, wherein the chicken is heterozygous for the modified IgH locus and the IgH locus on the homologous chromosome is wild type,, wherein the chicken is heterozygous for the modified IgH locus and the IgH locus on the homologous chromosome lacks its entire contiguous endogenous chicken V-D-J region, or wherein the chicken is heterozygous for the modified IgH locus and the IgH locus on the homologous chromosome lacks a J region.

11. The transgenic chicken of any prior claim, wherein the antibodies produced by the chicken are diversified in the heavy chain CDR1, CDR2 and CDR3 regions.

12. A B cell derived from the transgenic chicken of any prior claim.

13. A method for producing an antibody comprising a human variable domain and specifically binding to an antigen comprising:
(a) immunizing a transgenic chicken of any prior claim with an antigen; and
(b) obtaining from said chicken an antibody or antibodies that specifically binds to said antigen.

14. A method for producing the transgenic chicken according to claim 1 comprising:
(a) removing the entire contiguous endogenous chicken V-D-J region from the immunoglobulin heavy chain (IgH) locus of a chicken; and
(b) inserting into the locus a construct comprising, in operable linkage:
(i) an immunoglobulin heavy chain gene promoter;
(ii) a germline human VH segment comprising a coding sequence for a variable domain comprising FR1, CDR1, FR2, CDR2, and FR3 sequences;
(iii) a human D cluster;
(iv) a human J segment; and
(vi) a plurality of pseudogenes of structure FR1-CDR1-FR2-CDR2-FR3 that each comprise:
FR1, FR2 and FR3 sequences that are at least 95% identical to or identical to the FR1, FR2 and FR3 sequences in the functional human VH segment of (b)(ii); and
CDR1 and CDR2 coding sequences that differ from pseudogene to pseudogene:
wherein steps (a) and (b) are done in any order.

## Patentansprüche

1. Transgenes Huhn, wobei das transgene Huhn ein Genom umfasst, das einen modifizierten endogenen schwere-Immunglobulinkette(IgH)-Locus umfasst:
(a) dem die gesamte zusammenhängende endogene Huhn-V-D-J-Region fehlt und
(b) der in funktionsfähiger Verknüpfung Folgendes umfasst:
(i) einen Promotor eines schwere-Immunglobulinkette-Gens,
(ii) ein menschliches Keimbahn-V_{H}-Segment, das eine codierende Sequenz für eine variable Domäne umfasst, die FR1-, CDR1-, FR2-, CDR2- und FR3-Sequenzen umfasst,
(iii) ein menschliches D-Cluster,
(iv) ein einzelnes menschliches J-Segment,
(v) eine Mehrzahl von Sequenzen, die konstante Regionen codieren; sowie stromaufwärts des menschlichen Keimbahn-VH-Segments unter (b)(ii):
(vi) eine Mehrzahl von Pseudogenen der Struktur FR1-CDR1-FR2-CDR2-FR3, die jeweils Folgendes umfassen:
FR1-, FR2- und FR3-Sequenzen, die zu mindestens 95 % identisch mit oder identisch mit den FR1-, FR2- und FR3-Sequenzen in dem funktionellen menschlichen VH-Segment unter (b) (ii) sind, und
CDR1- und CDR2-Sequenzen, die sich von Pseudogen zu Pseudogen unterscheiden, wobei:
der modifizierte IgH-Locus in B-Zellen des Huhns einer VDJ-Rekombination unterliegt,
die Mehrzahl von menschlichen Keimbahn-V_{H}-Pseudogenen nach VDJ-Rekombination als Donoren mittels Genkonversion Nukleotidsequenzen an das menschliche Keimbahn-V_{H}-Segment unter (b) (ii) überträgt und
das Huhn humanisierte Antikörper produziert, die eine diversifizierte variable Domäne einer menschlichen schweren Immunglobulinkette umfassen.

2. Transgenes Huhn nach Anspruch 1, wobei die CDR1- und CDR2-Sequenzen unter (b) (vi), die die CDR1 und CDR2 verschiedener menschlicher VH-Segmente codieren, in derselben Familie sind wie das funktionelle menschliche VH-Segment unter (b) (ii).

3. Transgenes Huhn nach einem vorhergehenden Anspruch, wobei die gesamte zusammenhängende endogene Huhn-V-D-J-Region unter (a) eine Länge im Bereich von 10-12 kb aufweist.

4. Transgenes Huhn nach einem vorhergehenden Anspruch, wobei der Promotor unter (b) (i) ein Promotor eines schwere-Immunglobulinkette-Gens von Huhn ist.

5. Transgenes Huhn nach einem vorhergehenden Anspruch, wobei das menschliche Keimbahn-VH-Segment unter (b) (ii) und die CDR1- und CDR2-Sequenzen unter (vi) aus der V_{H}3-Familie, der V_{H}1-Familie oder der V_{H}4-Familie stammen.

6. Transgenes Huhn nach einem vorhergehenden Anspruch, wobei in dem D-Cluster sämtliche Codons für ein Cystein so mutiert sind, dass sie eine andere Aminosäure codieren, wobei optional in dem D-Cluster sämtliche Codons für ein Cystein so mutiert sind, dass sie Tyrosin oder Tryptophan codieren.

7. Transgenes Huhn nach einem vorhergehenden Anspruch, wobei die intervenierenden Sequenzen im D-Cluster von Huhn stammen.

8. Transgenes Huhn nach einem vorhergehenden Anspruch, wobei die Mehrzahl von Pseudogenen unter (b) (v) mindestens 10 der Pseudogene umfasst, wobei die Pseudogene unter (b) (v) in reverser Orientierung zu dem menschlichen Keimbahn-VH-Segment unter (b) (ii) liegen und/oder wobei die Mehrzahl von konstante Regionen codierenden Sequenzen unter (b)(v) für das Huhn endogen ist.

9. Transgenes Huhn nach einem vorhergehenden Anspruch, wobei das Transkript des modifizierten schwere-Immunglobulinkette(IgH)-Locus ein Intron umfasst, das das 3'-Ende einer Kopie der J codierenden Sequenz mit dem 5'-Ende einer Kopie der die konstante Region codierenden Sequenz unter (b) (v) verknüpft.

10. Transgenes Huhn nach einem vorhergehenden Anspruch, wobei das Huhn homozygot für den modifizierten IgH-Locus ist, wobei das Huhn heterozygot für den modifizierten IgH-Locus ist und der IgH-Locus auf dem homologen Chromosom ausgeknockt ist, wobei das Huhn heterozygot für den modifizierten IgH-Locus ist und der IgH-Locus auf dem homologen Chromosom Wildtyp ist, wobei das Huhn heterozygot für den modifizierten IgH-Locus ist und dem IgH-Locus auf dem homologen Chromosom dessen gesamte zusammenhängende endogene Huhn-V-D-J-Region fehlt oder wobei das Huhn heterozygot für den modifizierten IgH-Locus ist und dem IgH-Locus auf dem homologen Chromosom eine J-Region fehlt.

11. Transgenes Huhn nach einem vorhergehenden Anspruch, wobei die von dem Huhn produzierten Antikörper in den CDR1-, CDR2- und CDR3-Regionen der schweren Kette diversifiziert sind.

12. B-Zelle, die von dem transgenen Huhn nach einem vorhergehenden Anspruch herrührt.

13. Verfahren zur Herstellung eines Antikörpers, der eine menschliche variable Domäne umfasst und spezifisch an ein Antigen bindet, umfassend:
(a) Immunisieren eines transgenen Huhns nach einem vorhergehenden Anspruch mit einem Antigen und
(b) Gewinnen eines Antikörpers oder von Antikörpern aus dem Huhn, der bzw. die spezifisch an das Antigen bindet bzw. binden.

14. Verfahren zur Herstellung eines transgenen Huhns nach Anspruch 1, umfassend:
(a) Entfernen der gesamten zusammenhängenden endogenen Huhn-V-D-J-Region aus dem schwere-Immunglobulinkette(IgH)-Locus eines Huhns und
(b) Einfügen eines Konstrukts in den Locus, das in funktionsfähiger Verknüpfung Folgendes umfasst:
(i) einen Promotor eines schwere-Immunglobulinkette-Gens,
(ii) ein menschliches Keimbahn-VH-Segment, das eine codierende Sequenz für eine variable Domäne umfasst, die FR1-, CDR1-, FR2-, CDR2- und FR3-Sequenzen umfasst,
(iii) ein menschliches D-Cluster,
(iv) ein menschliches J-Segment und
(vi) eine Mehrzahl von Pseudogenen der Struktur FR1-CDR1-FR2-CDR2-FR3, die jeweils Folgendes umfassen:
FR1-, FR2- und FR3-Sequenzen, die zu mindestens 95 % identisch mit oder identisch mit den FR1-, FR2- und FR3-Sequenzen in dem funktionellen menschlichen VH-Segment unter (b) (ii) sind, und
CDR1 und CDR2 codierende Sequenzen, die sich von Pseudogen zu Pseudogen unterscheiden:
wobei die Schritte (a) und (b) in beliebiger Reihenfolge durchgeführt werden.

## Revendications

1. Poulet transgénique, le poulet transgénique comprenant un génome comprenant un locus de chaîne lourde d'immunoglobuline (IgH) endogène modifié qui :
(a) est dépourvu de la totalité de la région V-D-J endogène contiguë de poulet ; et
(b) comprend, en liaison opérationnelle :
(i) un promoteur génique de chaîne lourde d'immunoglobuline ;
(ii) un segment V_{H} humain de la lignée germinale comprenant une séquence codante pour un domaine variable qui comprend les séquences FR1, CDR1, FR2, CDR2 et FR3 ;
(iii) un cluster D humain ;
(iv) un seul segment J humain ;
(v) une pluralité de séquences codant pour des régions constantes ;
et, en amont du segment V_{H} humain de la lignée germinale de (b) (ii) :
(vi) une pluralité de pseudogènes de structure FR1-CDR1-FR2-CDR2-FR3 qui comprennent chacun :
des séquences FR1, FR2 et FR3 qui sont identiques ou au moins à 95 % identiques aux séquences FR1, FR2 et FR3 présentes dans le segment V_{H} humain fonctionnel de (b) (ii) ; et
des séquences CDR1 et CDR2 qui diffèrent d'un pseudogène à un autre :
le locus d'IgH modifié subissant une recombinaison VDJ dans les lymphocytes B du poulet ;
la pluralité de pseudogènes de V_{H} humain de la lignée germinale, après recombinaison VDJ, donnant des séquences nucléotidiques au segment V_{H} humain de la lignée germinale de b(ii) par conversion génique ; et
le poulet produisant des anticorps humanisés qui comprennent un domaine variable de chaîne lourde d'immunoglobuline humaine diversifié.

2. Poulet transgénique selon la revendication 1, dans lequel les séquences CDR1 et CDR2 de (b) (vi) qui codent pour les CDR1 et les CDR2 de différents segments V_{H} humains sont dans la même famille que le segment V_{H} humain fonctionnel de (b) (ii).

3. Poulet transgénique selon une quelconque revendication précédente, la totalité de la région V-D-J endogène contiguë de poulet de (a) ayant une longueur dans la plage de 10 à 12 kb.

4. Poulet transgénique selon une quelconque revendication précédente, le promoteur de (b) (i) étant un promoteur génique de chaîne lourde d'immunoglobuline de poulet.

5. Poulet transgénique selon une quelconque revendication précédente, le segment V_{H} humain de la lignée germinale de (b) (ii) et les séquences CDR1 et CDR2 de (vi) provenant de la famille V_{H}3, de la famille V_{H}1 ou de la famille V_{H}4.

6. Poulet transgénique selon une quelconque revendication précédente, de quelconques codons pour une cystéine, dans le cluster D, ayant été mutés pour coder pour un autre acide aminé et, éventuellement, de quelconques codons pour une cystéine, dans le cluster D, ayant été mutés pour coder pour la tyrosine ou le tryptophane.

7. Poulet transgénique selon une quelconque revendication précédente, les séquences intermédiaires présentes dans le cluster D provenant du poulet.

8. Poulet transgénique selon une quelconque revendication précédente, la pluralité de pseudogènes de (b) (v) comprenant au moins 10 des pseudogènes, les pseudogènes de (b) (v) ayant une orientation inverse par rapport au segment V_{H} humain de la lignée germinale de (b) (ii), et/ou la pluralité de séquences codant pour des régions constantes de (b) (v) étant endogènes au poulet.

9. Poulet transgénique selon une quelconque revendication précédente, le transcrit du locus de chaîne lourde d'immunoglobuline (IgH) modifié comprenant un intron qui relie l'extrémité 3' d'une copie de la séquence codante J à l'extrémité 5' d'une copie de la séquence codant pour une région constante de (b) (v).

10. Poulet transgénique selon une quelconque revendication précédente, le poulet étant homozygote pour le locus d'IgH modifié, le poulet étant hétérozygote pour le locus d'IgH modifié et le locus d'IgH sur le chromosome homologue étant inactivé, le poulet étant hétérozygote pour le locus d'IgH modifié et le locus d'IgH sur le chromosome homologue étant de type sauvage, le poulet étant hétérozygote pour le locus d'IgH modifié et le locus d'IgH sur le chromosome homologue étant dépourvu de la totalité de sa région V-D-J endogène contiguë de poulet ou le poulet étant hétérozygote pour le locus d'IgH modifié et le locus d'IgH sur le chromosome homologue étant dépourvu d'une région J.

11. Poulet transgénique selon une quelconque revendication précédente, les anticorps produits par le poulet étant diversifiés dans les régions CDR1, CDR2 et CDR3 de la chaîne lourde.

12. Lymphocyte B issu du poulet transgénique selon une quelconque revendication précédente.

13. Procédé de production d'un anticorps comprenant un domaine variable humain et se liant spécifiquement à un antigène comprenant :
(a) l'immunisation d'un poulet transgénique selon une quelconque revendication précédente avec un antigène ; et
(b) l'obtention à partir dudit poulet d'un ou plusieurs anticorps qui se lient spécifiquement audit antigène.

14. Procédé de production du poulet transgénique selon la revendication 1 comprenant :
(a) l'élimination de la totalité de la région V-D-J endogène contiguë de poulet du locus de chaîne lourde d'immunoglobuline (IgH) d'un poulet ; et
(b) l'insertion dans le locus d'une construction comprenant, en liaison opérationnelle :
(i) un promoteur génique de chaîne lourde d'immunoglobuline ;
(ii) un segment V_{H} humain de la lignée germinale comprenant une séquence codante pour un domaine variable comprenant les séquences FR1, CDR1, FR2, CDR2 et FR3 ;
(iii) un cluster D humain ;
(iv) un segment J humain ; et
(vi) une pluralité de pseudogènes de structure FR1-CDR1-FR2-CDR2-FR3 qui comprennent chacun :
des séquences FR1, FR2 et FR3 qui sont identiques ou au moins à 95 % identiques aux séquences FR1, FR2 et FR3 présentes dans le segment V_{H} humain fonctionnel de (b) (ii) ; et
des séquences codantes CDR1 et CDR2 qui diffèrent d'un pseudogène à un autre,
les étapes (a) et (b) étant effectuées dans n'importe quel ordre.
